# EUROPEAN PATENT SPECIFICATION

(11) **EP 1 147 179 B1**
(45) Date of publication and mention of the grant of the patent: **12.11.2008**
(21) Application number: 00906940.2
(22) Date of filing: 19.01.2000
(51) Int. Cl.: C12N 5/08, C12N 5/10, A61K 35/407, A01N 1/02, A61P 1/16, A61P 35/00

(54) **HUMAN LIVER PROGENITORS**
MENSCHLICHE LEBER-VORLÄUFERZELLEN
PROGENITEURS DE FOIE HUMAIN

(30) Priority: 19.01.1999 US 116331 P
(43) Date of publication of application: 24.10.2001
(73) Proprietor: THE UNIVERSITY OF NORTH CAROLINA AT CHAPEL HILL, Chapel Hill, NC 27599-4105 (US)
(72) Inventor: REID, Lola, M., Chapel Hill, NC 27514 (US); KUBOTA, Hiroshi, Chapel Hill, NC 27514-8668 (US); MOSS, Nicholas, Carrboro, NC 27510 (US)
(74) Representative: Luderschmidt, Schüler & Partner
(86) International application number: PCT/US2000/001116
(87) International publication number: WO 2000/043498

(56) References cited:
- EP-A- 0 834 252
- WO-A-95/13697
- US-A- 5 789 246
- MITAKA T ET AL: "GROWTH AND MATURATION OF SMALL HEPATOCYTES ISOLATED FROM ADULT RAT LIVER" BIOCHEMICAL AND BIOPHYSICAL RESEARCH COMMUNICATIONS,US,ACADEMIC PRESS INC. ORLANDO, FL, vol. 214, no. 2, 14 September 1995 (1995-09-14), pages 310-317, XP000197432 ISSN: 0006-291X
- AGELLI M ET AL: "ANTIGENIC CHARACTERIZATION OF PUTATIVE LIVER STEM CELLS OBTAINED FROM THE LIVER OF NORMAL ANIMALS" CLINICAL RESEARCH, vol. 40, no. 3, 1992, page 659A XP000938909 ISSN: 0009-9279
- TANIGUCHI H ET AL: "Characterization of hematopoietic stem cells in the adult liver." TRANSPLANTATION PROCEEDINGS, vol. 29, no. 1-2, 1997, pages 1212-1213, XP000938932 Sixteenth International Congress of the Transplantation Society;Barcelona, Spain; August 25-30, 1996 ISSN: 0041-1345
- M AGELLI ET AL: "Putative liver progenitor cells: conditions for long-term survival in culture" JOURNAL OF HISTOTECHNOLOGY,US,DOWNERS GROVE, IL, vol. 29, no. 29, 1997, pages 205-217, XP002123294 ISSN: 0147-8885

## Description

### Field of The Invention

The present invention relates to a method of providing a composition comprising a mixture of cells derived from human liver tissue, which mixture comprises an enriched population of human liver progenitors. These human liver progenitors are human hepatic stem cells, pluripotent cells that give rise to hepatocytes and biliary cells, and other liver progenitor cell subpopulations that have the capacity to expand and differentiate into one or more liver cell lineages including hemopoietic, mesenchymal or hepatic cell lineages. In particular, the invention relates to markers and properties used to identify human liver progenitors, methods of their purification, novel approaches that enable one to distinguish hepatic from hemopoietic subpopulations, and evidence proving that hepatic progenitors exist in livers from fetal to adult human livers. The inventions constitute the basis for cell and gene therapies and for the establishment of bioartificial organs.

### Background

The primary structural and functional unit of the mature liver is the acinus, which in cross section is organized like a wheel around two distinct vascular beds: 3-7 sets of portal triads (each with a portal venule, hepatic arteriole, and a bile duct) for the periphery, and with the central vein at the hub. The liver cells are organized as cell plates lined on both sides by fenestrated endothelia, defining a series of sinusoids that are contiguous with the portal and central vasculature. Recent data have indicated that the Canals of Hering, small ducts located around each of the portal triads, produce tiny ductules that extend and splice into the liver plates throughout zone 1 forming a pattern similar to that of a bottle brush (Theise, N. 1999 Hepatology. 30: 1425-1433).

A narrow space, the Space of Disse, separates the endothelia from hepatocytes all along the sinusoid. As a result of this organization, hepatocytes have two basal domains, each of which faces a sinusoid, and an apical domain which is defined by the region of contact between adjacent hepatocytes. The basal domains contact the blood, and are involved in the absorption and secretion of plasma components, while the apical domains form bile canaliculi, specialized in the secretion of bile salts, and are associated through an interconnecting network with bile ducts. Blood flows from the portal venules and hepatic arterioles through the sinusoids to the terminal hepatic venules and the central vein.

Based on this microcirculatory pattern, the acinus is divided into three zones : zone 1, the periportal region ; zone 2, the midacinar region, and zone 3, the pericentral region. Proliferative potential, morphological criteria, ploidy, and most liver-specific genes are correlated with zonal location (Gebhardt, R., et al. 1988. FEBS Lett. 241 : 89-93; Gumucio, J. J. 1989, Vol. 19. Springer International, Madrid ; Traber, P. et al. 1988. Gastroenterology. 95: 1130-43). Gradients in the concentration of blood components, including oxygen, across the acinus, and following the direction of blood flow from the portal triads to the central vein, are responsible for some of this zonation, for example the reciprocal compartmentation of glycolysis and gluconeogenesis. However, the periportal zonation of the gap junction protein connexin 26 and the pericentral zonation of glutamine synthetase, to name only two, are insensitive to such gradients, are more representative of most tissue-specific genes and appear to be determined by factors intrinsic to the cells or to variables other than blood flow in the microenvironment.

In addition to hepatocytes, bile duct epithelial cells (cholangiocytes), and endothelial cells, the region between the portal and central tracts contains other cell types, such as Ito cells and Kupffer cells. These play prominent roles in pathogenic conditions of the liver, especially in inflammation and fibrosis, but their direct contribution to the main homeostatic functions of the normal organ are apparently small.

The liver develops as a result of the convergence of a diverticulum formed from the caudal foregut and the septum transversum, part of the splanchnic mesenchyme. The formation of the hepatic cells begins after the endodermal epithelium interacts with the cardiogenic mesoderm, probably via fibroblast growth factors. The specified hepatic cells then proliferate and penetrate into the mesenchyme of the septum transversum with a cord like fashion, forming the liver anlage. The direct epithelial-mesenchymal interaction is critical in these early developmental stages of the liver and dictates which cells will become hepatocytes or cholangiocytes, and the fenestrated endothelia, respectively. Mutations in the mesenchyme-specific genes hlx and jumonji block liver development, illustrating the importance of contributions from this tissue. Early in its development, the liver consists of clusters of primitive hepatocytes bounded by a continuous endothelium lacking a basement membrane and abundant hemopoietic cells. As the endothelium is transformed to become a discontinuous, fenestrated endothelium, the vasculature, especially the portal vasculature, becomes more developed with the production of basement membranes. The portal interstitium may provide the trigger for the development of bile ducts, and as it surrounds the portal venules, hepatic arterioles, and bile ducts, portal triads are formed. Immature hepatocytes rapidly proliferate and parenchymal plates are formed, probably in response to changes in the amount and distribution of such tissue-organizing molecules as C-CAM 105, Agpl 10, E-cadherin, and connexins, coincident with the relocation of most, but not all, of the hemopoietic cells to the bone marrow. Recent studies suggest that some hemopoietic progenitors persist in the adult quiescent rodent liver, and hemopoietic stem cells have been isolated from both adult human and murine liver (Crosbie, O. M. et al. 1999. Hepatology. 29: 1193-8). The mature physical organization is achieved within the first weeks after birth in rodents, and in humans, within the first few years. Metabolic zonation is established according to somewhat different schedules for different enzymes, but becomes evident in the period following birth.

### Stem cells and committed progenitors

Stem cells have been defined as primitive cells that self-replicate, that are pluripotent, i.e. produce daughter cells with more than one fate, that can expand extensively and can reconstitute a tissue or tissues. Most of the literature on stem cells derives either from the literature on embryos or that on hemopoietic, epidermal, or intestinal tissues.

More recently, the definitions have been modified to recognize particular classes of stem cells. Those with the potential to participate in the development of all cell types including germ cells are referred to as ***totipotent stem cells*** and include the zygote and normal embryonic cells up to the 8 cell stage (the morula). Embryonic stem cells, also called "ES"cells, consist of permanent cell populations derived from totipotent, normal cells in blastocysts, that were first reported in the early 1980s. ES cell lines can be cultured in vitro with maintenance of totipotency. ES cells are tumorigenic if introduced into immunocompromised hosts in any site other than in utero, forming teratocarcinomas. However, when they are injected back into normal blastocysts, they are able to resume embryonic development and participate in the formation of a normal, but chimeric, mouse. Although ES cell lines have been established from many species (mouse, rat, pig, etc.), only the mouse system has been used routinely to generate animals with novel phenotypes (knockouts, transgenics) by merging modified ES cells from culture to blastocysts and then implanting the blastocysts into pseudopregnant hosts. Embryonic germ (EG) cell lines, which show many of the characteristics of ES cells, can be isolated directly in vitro from the primordial germ cell population. As with ES cells, the EG cells form teratocarcinomas when injected into immunocompromised mice and contributed to chimeras, including the germ line, when injected into blastocysts.

*Determined stem cells* are pluripotent cells that have restricted their genetic potential to that for a limited number of cell types and have extensive growth potential. Increasing evidence such as that from the telomerase field suggest that determined stem cells do not self- replicate, that is their progeny can have less growth potential than the parent. Determined stem cells give rise to daughter cells that lose pluripotency by restricting their genetic potential to a single fate, *e.g*. hepatocytes, and are referred to as committed progenitors. In the hepatic lineage there are committed hepatocytic progenitors and committed biliary progenitors.

Recent, highly publicized experiments have reported that human ES cell cultures can be established from human embryos. It has been suggested that these human ES cells may be injected into tissues in the hope that they will be able to reconstitute damaged organs and tissues. Given the findings that ES and EG cells form tumors when injected into sites other than in utero (see above), the plan to inoculate human ES cells into patients is unrealistic and with the grave possibility of creating tumors in the patients. To overcome this impasse, some groups are pursuing the plan of differentiating the ES cells under defined microenviron-mental conditions to become determined stem cells that can then be safely inoculated into patients. For example, there is some measure of success in generating hemopoietic progenitors. However, the concern remains that residual ES cells in the culture could pose the risk of tumorigenesis, if the cultures are inoculated into a patient. In summary, until research in developmental biology reveals the myriad controls dictating the fates of cells during embryogenesis, the ES cells will remain as an experimental tool with little hope for clinical programs in cell or gene therapies. The only realistic option for clinical programs in cell and gene therapies is to use determined stem cells in which the genetic potential is restricted to a limited number of cell types. By contrast, the ES cells may hold great promise for bioartificial organs for those tissue types (*e.g*. hemopoietic cells) that are produced by ES cells under known conditions.

### Controversy surrounding liver stem cells

The presence of stem cells in adult normal liver is the subject of great controversy in the field of liver cell biology. Below are summarized the several prevailing models competing in the field. The italicized text indicates the key idea of the different models.

It is believed by some experts in the field that ***hepatic stem cells exist only in*** embryonic tissue, that there are no stem cells in adult livers, and that all mature liver cells participate equally in liver regenerative processes (Farber, E. 1992. In The Role of Cell Types in Hepatocarcinogenesis. S. A. E, editor. Academic Press, New York.). The Farber model considers all mature parenchymal cells to be phenotypically co-equal and that the known heterogeneity of growth potential and gene expression in liver is due only to microenvironment. Farber proposes that under oncogenic conditions, adult parenchymal cells retro-differentiate and become tumor cells. This model dominated the liver carcinogenesis field for decades and still has impact in liver regeneration studies.

Other experts believe that all liver cells are stem cells (Kennedy, S. et al. 1995. Hepatology. 22: 160-8 ; Michalopoulos, G. K. et al. 1997, Science. 276: 60-6.). These investigators believe that all parenchymal cells are co-equal, are highly plastic and with gene expression dictated only by the microenvironment. Under appropriate oncogenic conditions, the mature parenchymal cells are hypothesized to become stem cells that can subsequently convert to tumor cells.

The **silent stem cell model is** based on the studies of Wilson and Leduc (Wilson, J. W. et al. 1958. J. Pathol. Bacteriol. 76: 441-449.). As in the hemopoietic field, this concept gained the most credibility from extensive studies of liver carcinogenesis (Marceau, N. 1994. Gut. 35 : 294-6.). These investigators believe that progenitor cells, including bipotential progenitor cells, can persist in adult tissue but propose that they are rare holdovers or remnants of cell populations from embryonic development. They assume that progenitors play no role in normal or regenerative liver functioning but only in disease states (Overturf K, et al. 1999. American Journal of Pathology. 155: 2135-2143.). That is, they are presumed to be "silent," similar to the satellite cells in muscle. These cells have been described as "oval cells" on account of the distinctive shape of the cell nuclei. They are small (∼9 um) and express a characteristic antigenic profile on the cell surface. All mature liver cells are assumed to be co-equal with respect to growth and gene expression and that all aspects of heterogeneity of gene expression is dictated only by the cellular microenvironment. The proponents of the silent stem cell model strongly reject any idea of movement of parenchymal cells from periportal to pericentral locations. The importance of stem cells and other hepatic progenitors is thought to be relevant to disease states only, especially carcinogenesis. Thus, these investigators have focused their efforts on candidate progenitors in animals treated with various oncogenic insults. These studies show that "oval cells" do not form a recognizable body of rapidly proliferating cells under regenerative conditions or under conditions of mild to moderate injuries. Significant numbers of proliferating oval cell populations are observed only after quite severe liver injuries, (Grisham, J. W. et al. 1997. In Stem Cells. C. S. Potter, editor. Academic Press, London. 233-282.).

A model based on *streaming of liver cells* (Arber, N. et al. 1988. Liver. 8: 80-7 ; Zajicek, G. et al. 1991. Liver. 11: 347-51) has been sharply criticized and largely ignored (Jirtle, R. L. 1995. Liver Regeneration and Carcinogenesis: Molecular and Cellular Mechanisms. Academic Press, New York.). This proposal postulates that a stem cell compartment at each of the portal triads yields adult parenchymal cells that "stream" towards the central vein. The streaming process brings the daughter cells into contact with distinct microenvironments resulting in changes in the phenotype of the cells. Again, the microenvironment is hypothesized to be the critical determinant of phenotype. A majority of investigators have argued against this model suggesting that it is inconsistent with studies showing no movement of marked donor cells reintroduced into liver (Kennedy, S. et al. 1995. Hepatology. 22 : 160-8.). However, even in studies that have provided the most definitive evidence countering the streaming model, it is unknown if the microenvironment or lineage position influences the expression of markers used in donor cells. Moreover, the streaming liver hypothesis is likely to be revisited after the recent findings by Thiese and his associates (Theise, N. 1999. Hepatology. 30 : 1425-1433.) that the Canals of Herring, long suspected of being related to hepatic progenitors, extend ductules throughout the liver plate at least in zone 1.

Reid and associates have advocated that the liver is ***a stem cell and maturational lineage*** ***system*** (Sigal, S. H. et al. 1992. Am JPhysiol. 263 : G 139-48.). They propose that tissues are organized as maturational lineages fed, like a spring, by stem cells or early progenitor cell populations (Brill, S. et al. 1993. Proceedings of the Society for Experimental Biology & Medicine. 204 : 261-9.). The tissue is defined as going from "young, to middle age, to old cells". The maturational process is accompanied by lineage-position-dependent changes in cell size, morphology, antigenic profiles, growth potential and gene expression. These changes are hypothesized to be due to a combination of autonomous cellular changes, independent of microenvironment, and of microenvironmentally induced changes; the microenvironment comprises the nutrients, gas exchange (oxygen, C02), pH, hormones, cell- cell interactions and extracellular matrix chemistry.

**Table 1.**

| **Zones** | **1** | **2** | **3** |
|---|---|---|---|
| **Polidy** | Diploid cells | Tetraploid cells | Mix of tetraploid and octaploid cells |
| **Average Size** | 7-20µ | 20-30µ | 30-50µ |
| **Growth** | Maximum | Intermediate | Negligible |
| **Extracellular Matrix** | A gradient in the matrix chemistry located in the space of Disse and consisting of type IV collagen mixed with laminin and heparin sulfate proteoglycans in the periportal area and converting to fibrillar collagens, fibronectins and heparin proteogylcns in the pericentral area | | |
| **Gene Expression** | Early | Intermediate | Late |

Growth is hypothesized to be maximal in the stem cells and early progenitors and to wane with progression through the lineage. This model takes into account that the majority of the cells in the adult liver tissue are polyploid, mostly tetraploid or octaploid, less than a third of the cells are diploid. Recent data support the concept that the bulk of the regenerative potential in a tissue derives from the diploid cell population and that the older cells contribute to regeneration by increasing cell mass via hypertrophic responses associated with polyploidy. (Sigal, S. H. et al. 1999. American Journal of Physiology. 276: G1260-72.). Therefore, these researchers advocate that the best hopes for cell growth, whether in cell or gene therapies or in bioartificial organs, is with the diploid cell population of the tissue.

The stem cell and maturational lineage model contradicts other liver cell development models in suggesting that liver malignancy is most often an indirect, rather than a direct, result of an oncogenic insult. Oncogenic insults are proposed to kill most cells of the liver, specially the mature cells in the lineage, resulting in a dramatic induction of a regenerative response. The resultant expansion of the progenitors increases the risk of secondary mutational events in the rapidly growing cells, the progenitors, that can result in malignancy. Thus, the older hypotheses that cancer is blocked differentiation or that cancers are due to oncogenic insults targeting stem cells are accepted as correct but with the modification presented above.

Increasing acceptance of a maturational lineage model is now based on the data that liver is replete with features indicative of an apoptotic or terminal differentiation process (Sigal, S. H. 1995. Differentiation. 59: 35-42.) and the findings that only certain subpopulations of liver cells present in adult livers are capable of extensive cell division (Overturf K, et al. 1999. American Journal of Pathology. 155 : 2135-2143 ; Tateno, C et al. 2000. Hepatology. 31: 65-74.). In this model the progenitors and a subpopulation of adult cells (presumed to be the diploid subpopulation) are capable of reconstituting liver tissue when re-injected *in vivo,* and are capable of extensive growth including clonal growth.

U. S. Patent No 5, 559, 022 to Naughton discloses isolation of cells from liver and further purification by the use of gradient centrifugation. However, the cell population isolated is the "acidophilic parenchymal cell population" which is not the liver progenitors of this invention as claimed.

### Pre-Clinical and Clinical Applicability of Liver Progenitors

There is a strong clinical and commercial interest in isolating and identifying immature progenitor cells from liver because of the impact that such cell population may have in treating liver diseases. Each year in the United States, there are about 250, 000 people hospitalized for liver failure. Liver transplants are curative for some forms of liver failure, and approximately 4100 transplants are performed a year in United States. One of the limiting factors in liver transplantation is the availability of donor livers especially given the constraint that donor livers for organ transplantation must originate from patients having undergone brain death but not heart arrest. Livers from cadaveric donors have not been successful, although recent efforts to use such donors have supported the possibility of using them if the liver is obtained within an hour of death.

Cell transplantation into the liver is an attractive alternative therapy for most liver diseases. The surgical procedures for cell transplantation are minor relative to those needed for whole organ transplantation and, therefore, can be used for patients with various surgical risks such as age or infirmity. The use of human liver cells is superior to liver cells derived from other mammals because the potential pathogens, if any, are of human origin and could be better tolerated by patients and could be easily screened before use.

Attempts to perform liver cell transplantation have made use of unfractionated mature liver cells and have shown some measure of efficacy (Fox, I. J. et al. 1998. New England Journal of Medicine. 338 : 1422-1426.). However, the successes require injection of large numbers of cells (10-20 billion), since the cells do not grow *in vivo.* Furthermore, the introduction of substantial numbers of large mature liver cells (average cell diameter 30-50µ) is complicated by their tendency to form large aggregates upon injection, resulting in potentially fatal emboli. Moreover, these cells elicit a marked immunological rejection response forcing patients to be maintained on immunosuppressive drugs for the remainder of their lives. Finally, mature liver cells have not been successfully cryopreserved and complicated logistics are required to coordinate the availability of suitable liver tissue, the preparation of cell suspensions and the immediate delivery of the cells for clinical therapies.

### Advances In Isolation of Liver Progenitors

Isolation of liver progenitors from liver is known to be an extremely challenging task due to the shortage of markers that positively select for liver cells. The only available antibodies for candidates of hepatic progenitors are those monoclonal antibodies that are prepared against subpopulations of hepatic progenitors (oval cells) induced to proliferate after exposure to oncogenic insults. These antibodies however cross-react with antigens present in hemopoietic cells.

Attempts have been made in the past to obtain the hepatic progenitor cell population, suggested to be the most versatile population for cell and gene therapy of the liver. U.S. Pat. Nos 5, 576, 207; 5, 789, 246 to Reid et al*.* utilize cell surface markers and side scatter flow cytometry to provide a defined subpopulation in the liver. Subpopulations of rat hepatic cells have been isolated by removal of lineage-committed cells followed by selection for immature hepatic precursors which were detected as being agranular cells bearing OC. 3-positive (oval cell antigenic marker), AFP-positive, albumin-positive, and CK19-negative (cytokeratin 19) cell markers. The foregoing rat liver subpopulations demonstrate particular characteristics important in isolation and identification of enriched hepatic progenitors from rodent liver.

Isolation of liver progenitors from adult human liver, as disclosed herein, is novel and unexpected partly due to the controversy regarding the mere presence of liver progenitors in the adult in which human hepatic progenitors have been assumed either not to be present or to be a physiologically silent remant from embryogenesis. Therefore, there have not been attempts to isolate them or study them except in disease states.

By way of contrast, within the developing liver the presence of the cytoplasmic proteins alpha-fetoprotein (AFP) and albumin is recognized as a strong positive indicator of progenitor cells. In the earliest stages of liver development these cells are capable of producing offspring that enter both biliary and hepatocyte lineages. If these daughter cells commit to the biliary lineage alpha-fetoprotein expression ceases. However, alpha-fetoprotein expression persists in the hepatocyte lineage until the perinatal period when it is suppressed, leaving albumin expression as one of the principal characteristics of the adult hepatocyte.

However, since alpha-fetoprotein is an intracellular protein and can only be visualized after fixation and permeabilization of the cell, it is unsuitable as a marker for the identification of viable hepatic progenitor cells.

### Summary of the Invention

The invention relates to a method of providing a composition comprising a mixture of cells derived from human liver tissue, which mixture comprises an enriched population of human hepatic progenitors, the method comprising: (a) providing a substantially single cell suspension of extracted human liver tissue; (b) debulking the suspension based on cell size, buoyant density, or a combination thereof to remove mature cells while retaining immature cells; and (c) subjecting the debulked suspension to a positive immunoselection comprising selecting those cells which express CD14, CD34, CD38 or combinations thereof, and/or a negative immunoselection comprising removing those cells which express CD45, glycophorin A or a combination thereof, such that a mixture of cells is provided, which mixture of cells is comprised of an enriched population of human hepatic progenitors and expresses alpha-fetoprotein, albumin, or both. The alpha-fetoprotein and albumin can be full-length or a variant. The debulking process can comprise a separation by cell size, buoyant density, or both. The debulking can also be based on sedimentation velocity, hydrodynamic radius, and sedimentation to equilibrium density. Alternatively, the separation can be by relative adherence of surface markers to binding components, for example antibodies or lectins. The isolated progenitors can be diploid and can be less than about 15 microns in diameter. Furthermore, the progenitors can synthesize macromolecules characteristic of progenitors, including, but not limited to alpha-fetoprotein and albumin. Preferably, the alpha- fetoprotein includes the exonl (aFP)-encoded peptide sequence. Thus the alpha-fetoprotein is transcribed from an mRNA greater than 2Kb in size, a full-length aFP mRNA. Likewise, the albumin preferably includes the exonl (ALB)-encoded peptide sequence. Thus the albumin is transcribed from a full-length mRNA.

In one embodiment of the invention, the liver tissue is obtained from a neonate, an infant, a child, a juvenile, or an adult.

In another embodiment of the invention, the enriched population comprises human diploid liver cells.

In a further embodiment of the invention, the debulking step comprises centrifugal elutriation, density gradient centrifugation, panning, affinity chromatography, tagging with fluorescent labels, countercurrent fluid flow, continuous-flow centrifugation, zonal centrifugation, use of magnetic beads, or combinations thereof.

In another embodiment, the invention comprises selective lysis of the mature cells.

In one embodiment of the invention, the immature cells have a diameter between 5 and 15 microns.

In another embodiment of the invention, the immature cells have a diameter between 8 and 9.4 microns.

The present invention relates to a method of isolation of progenitors from human liver which includes processing human liver tissue to provide a substantially single cell suspension including progenitors and non- progenitors of one or more cell lineages found in human liver; subjecting the suspension to a debulking step, which reduces substantially the number of non-progenitors in the suspension, to provide a debulked suspension enriched in progenitors exhibiting one or more markers associated with at least one of the cell lineages; selecting from the debulked suspension those cells, which express at least one marker associated with at least one liver cell lineage, i. e. CD14, CD34, CD38 or combinations thereof . Processing or debulking steps of this invention preferably include a density gradient centrifugation or centrifugal elutriation of the liver cell suspension to separate the cells according to their buoyant density and/or size, which are associated with one or more gradient fractions having a lower buoyant density and/or smaller size. The density gradient method can include zonal centrifugation and continuous-flow centrifugation.

One embodiment of the invention is negative selection of non-progenitors including mature hepatic, hemopoietic, and mesenchymal cells by the use of markers associated with mature hepatic cells, markers associated with hemopoietic cells, such as glycophorin A and CD45 .

The inventors have found that use of hepatic progenitors can overcome many of the shortcomings associated with use of mature liver cells, making them ideal cells for use in cell and gene therapies and for bioartifical organs. The cells are small (7-15 µ), therefore minimizing the formation of large emboli. Also, the cells have extensive growth potential meaning that fewer cells are needed for reconstitution of liver tissue in a patient. Finally, the progenitors have minimal antigenic markers that might elicit immunological rejection providing hope that little or no immunosuppressive drugs might be needed. Therapy with liver cells involves either extracorporeal treatment or transplantation of liver cells. The cells, preferably including progenitor cells, are supplied in any of various ways, including parenterally and intraperitoneally. An effective amount of cells is necessary, preferably between 103 and 10 10 cells. More preferably between 105 and 108 cells are transplanted, optimally about 106 cells.

Liver progenitors are extremely useful for production of growth factors and other proteins. These factors are associated with their own growth or that of other progenitors in the liver (*e.g.* hemopoietic or mesenchymal progenitors) and factors associated with early steps in the dedication of hepatic progenitor cells to a particular lineage. These novel growth factors can be used to treat liver disease or to control those cancers that are transformants of the liver progenitors. Furthermore, liver progenitors are important targets for gene therapy, wherein the inserted genetically transformed or normal hepatic progenitors promote the health of the individual into whom such hepatic progenitors are transplanted.

Disclosed herein is the determination of unique antigenic profiles on the cell surface that correlate with the expression of alpha-fetoprotein within the cell. Characterization of alpha-fetoprotein-containing cells in this way allows the subsequent enrichment of viable hepatic progenitor cells by flow cytometric methodology from living single cell suspensions prepared from whole livers or liver lobes. Moreover, the isolation and identification of human hepatic progenitors as described herein were obtained through application of a combination of unique methods, markers and parameters which the present inventors used for the first time to achieve the unique cell population of this invention.

A further aspect of this invention provides for liver cell progenitors of hepatic, hematopoietic, or mesenchymal origin. These cell lineages are selected by antigenic markers selected from the group consisting of CD14, CD34, CD38, CD45, and/or glycophorin A, and express cytoplasmic markers such as alpha-fetoprotein, albumin, or both. Alpha-fetoprotein can derive from a full-length mRNA (greater than 2 Kb, the form usually expressed in hepatic progenitors) or from a variant form (less than 2 Kb, i.e. approximately 0.5, 0.8, 1, 1.5, or 2 Kb, the form usually expressed in hemopoietic progenitors). The liver progenitors of this invention can be isolated from the liver of a fetus, a neonate, an infant, a child, a juvenile, or an adult.

In accordance with yet a further aspect of this invention, isolated human liver progenitors are isolated in a highly enriched to substantially pure form. Such liver progenitors contain hepatic, hemopoietic and mesenchymal progenitors. The hepatic progenitors have the capacity to develop into hepatocytes, biliary cells, or a combination thereof; the hematopoietic progenitors have the capacity to develop into macrophages, neutrophils, granulocytes, lymphocytes, platelets, neutrophils eosinophils, basophils, or a combination thereof. The mesenchymal progenitors have the capacity to develop into endothelial cells, stromal cells, hepatic stellate cells (Ito cells), cartilage cells, bone cells or combinations thereof.

The liver progenitors can harbor exogenous nucleic acid. Such exogenous nucleic acid can encode at least one polypeptide of interest, or can promote the expression of at least one polypeptide of interest.

The negative effects of one or more human disorders or dysfunctions can be alleviated by administering to an individual suffering from such negative effects an effective amount of isolated human liver progenitors. The progenitors can be administered either intraperitoneally, or parenterally via a vascular vessel, or administered directly into the liver. The direct administration may be effected surgically via portal vein, mesenteric vein, hepatic artery, hepatic bile duct, or combinations thereof. Alternatively, the liver progenitors can be administered into an ectopic site of the individual, such as spleen or peritoneum.

The human disorders or dysfunctions that can be alleviated by the administration of the isolated human liver progenitors disclosed herein include: hepatocholangitis, hepatomalacia, hepatomegalia, cirrhosis, fibrosis, hepatitis, acute liver failure, chronic liver failure, or inborn errors of metabolism, and liver cancer such as hepatocarcinoma, or hepatoblastoma. The cancer of the liver can be a primary site of cancer or one that has metastasized into the liver. The metastatic tumor could be derived from any number of primary sites including, intestine, prostate, breast, kidney, pancreas, skin, brain, lung or a combination thereof.

Furthermore, a bioreactor can include biological material comprising isolated progenitors from human liver; and culture media, such as basal media; one or more compartments holding the biological material or the components comprising the biological material; and optionally one or more connecting ports. Furthermore the bioreactor can, optionally, also include: extracellular matrix; hormones, growth factors, nutrients, or combinations thereof; and a biological fluid such as serum, plasma, or lymph.

The bioreactor is adapted for sustaining said progenitors in a viable, functional state, and can sustain liver progenitors for, a period ranging from about one week to about 55 weeks. Specifically, the bioreactor is adapted for use as an artificial liver, for product manufacturing, toxicological studies, or metabolic studies, including studies involving the activity of cytochrome P450, or other types of drug metabolism.

Disclosed herein is a composition of isolated human liver progenitors, or a suspension enriched in progenitors obtained from human liver is provided. The cell suspension can be provided in a pharmaceutically acceptable carrier or diluent and is administered to a subject in need of treatment. The composition disclosed includes liver progenitors that exhibit one or more markers associated with at least one of one or more cell lineages found in human liver and are substantially free of mature cells. More particularly, isolated liver progenitors are derived from one or more liver cell lineages including hepatic, hemopoietic, or mesenchymal cell lineages and express at least one or more of antigenic markers CD 14, CD34, CD38 and cytoplasmic markers of alpha-fetoprotein, albumin, or both. In a further embodiment, the immature cells, their progeny, or more mature forms express osteopontin, bone sialoprotein, collagen I, collagen III, collagen IV, or a combination thereof.

A cell culture system of liver progenitors can include isolated progenitors from human liver. The cell culture system additionally includes extracellular matrix comprising one or more collagens, one or more adhesion proteins (laminins, fibronectins), and other components such as proteoglycans (such as heparan sulfate proteoglycans); or an individual matrix component. The matrix component includes fragments of matrix components, matrix mimetics that can be synthetic and biodegradable materials (i. e. microspheres) coated with one or more of the factors from one of the classes of extracellular matrices. The cell culture system additionally can include basal or enriched media and other nutrients; hormones, growth factors, and, optionally, a biological fluid such as serum, plasma or lymph. Additionally, the cell culture system can have one or more compartments that holds the biological material such as a culture dish, plate, flask, roller bottle, transwell or other such container.

The cultures or bioreactors can be used in one or more metabolic studies including studies involving the activity of phase I or II biotransformation enzyme systems, one or more transport studies including studies involving the expression, regulation and activity of hepatic sinusoidal and canalicular transport systems, facets of drug metabolism, and the activity of cytochrome P450 among others.

A method for cryopreservation of adherent cells can comprise (a) providing adherent cells and a matrix or a viscosity enhancer; (b) suspending the cells in a cryopreservation mixture comprising culture medium, an ice-crystal inhibitor, a carbohydrate regulating factor, an iron donator, a lipoprotein, and a lipid; and (c) cooling the suspension to below the freezing point of the cells. The freezing point here means the temperature at which the cells become a solid mass, whether that is a supercooled liquid or glass, a microcrystalline or macrocrystalline mass. Moreover, a mixture for cryopreservation is disclosed that comprises a culture medium, an ice-crystal inhibitor, a carbohydrate regulating factor, an iron donator, a lipoprotein, and a lipid. The cryopreservation mixture can also include an antioxidant, such as ascorbic acid, glycerol (10% v/v) or dimethylsulfoxide (DMSO, 10% v/v), the latter two agents which can act as inhibitors of ice crystal formation. The carbohydrate- regulating factor can be insulin or insulin-like growth factor. The iron donator, lipoprotein, and lipid can be transferrin, high density lipoprotein, and free fatty acids, respectively. The free fatty acids are optionally complexed with albumin. The cryopreservation mixture can include collagen, a collagen-like substance, agarose, methylcellulose, or gelatin, where the collagen can be collagen I, collagen, III, or collagen IV. The components of the cryopreservation mixture can be prepared in Viaspan or University of Wisconsin cryopreservation solution.

Furthermore, a collection, cell bank, catalog or biologic repository can have a plurality of cryopreserved hepatic progenitors. The progenitors can be isolated by the method described above Therefore, the progenitors can express markers indicative of expression of full-length alpha-fetoprotein, albumin, or both. The repository can include a system of indexing of cell markers. Upon thawing, the cells of the repository can be used to inoculate bioreactors, to initiate cell cultures, or for therapy of patients.

The term alpha-fetoprotein as used herein comprises a variant alpha-fetoprotein which is the gene product of a gene or mRNA missing exonl, defined below. As disclosed in this invention, the variant alpha-fetoprotein is often associated with hemopoietic progenitors and their progeny and not associated with hepatic progenitors.

A three to ten amino acid peptide can be taken from the alpha-fetoprotein exon 1-encoded sequence.

A conjugate of a macromolecule and a peptide comprising between three and ten amino acids from the alpha-fetoprotein exon 1- encoded sequence can be suitable for use as an antigen. The macromolecule can be albumin, hemocyanin, casein, ovalbumin, polylysine, *e.g*. poly-L-lysine or poly-D-lysine, and any other suitable macromolecule known in the art. The antigen can be used generate antibodies specific for the alpha-fetoprotein whose expression is indicative of hepatic progenitors and not indicative of hemopoietic progenitors or their progeny. The antibodies can be produced by immunizing an animal with the antigen in the absence or presence of adjuvant, or by exposing spleen cells to the antigen followed by fusion of the spleen cells to form hybridomas, as is known in the art.

In another embodiment of the invention a method for isolating progenitors from human liver is disclosed, comprising processing human liver tissue to provide a substantially single cell suspension comprising progenitors and non-progenitors of one or more cell lineages found in human liver, subjecting the suspension to a debulking step, which reduces substantially the number of non-progenitors in the suspension to provide a debulked suspension enriched in progenitors exhibiting one or more markers associated with at least one of the one or more cell lineages, and selecting from the debulked suspension those cells, which express one or more markers mentioned above which are associated with at least one of the one or more cell lineages.

### Brief Description of the Figures

Figure 1. PCR Analysis of alpha-Fetoprotein mRNA
Figure 2. PCR Analysis of Albumin mRNA
Figure 3. Effect of Cryopreservation on Fetal Liver Cell Viability
Figure 4. Left panel, Histogram of alpha-Fetoprotein Immunofluorescence by FACS Right panel, Histogram of Albumin Immunofluorescence by FACS
Figure 5. Percent of cells Expressing Surface Markers CD14, CD34, CD38, CD45, and Glycophorin A (GA) in Unfractionated Liver Cell Suspensions.
Figure 6. Coexpression of Cell Surface Markers and alpha-Fetoprotein by Fetal Liver Cells
Figure 7. Top left, Percent of Cells Positive for alpha-Fetoprotein Top right, Percent of cells Positive for Albumin Bottom, Effect of Percoll Fractionation on alpha-Fetoprotein and Albumin Coexpression
Figure 8. FACS Analysis of a Fetal Liver Cell Suspension for Co-Expression of CD14, CD38 and alpha-Fetoprotein
Figure 9. Yield of alpha-Fetoprotein-positive cells using selection with CD 14 and/or CD 38.
Figure 10. Four Representative Immunofluorescence views of Fetal Hepatic Progenitor Cells Stained for alpha-Fetoprotein.
Figure 11. Effect of Selection for CD14 (right) : Differential Interference Contrast (top) and Immunofluorescence Views (bottom).
Figure 12A. A cluster of Liver Cells by Phase Contrast Microscopy.
Figure 12B. The same cluster of Liver Cells by Immunofluorescence with antibody to alpha- Fetoprotein.
Figure 12C. An overlay of A and B.
Figure 13A. Liver Cells Stained with Calcein.
Figure 13B. Liver Cells Stained with alpha-Fetoprotein, same view as panel A.

### Detailed Description of Preferred Embodiments

### I. Definitions

In the description that follows, a number of terms are used extensively to describe the invention. In order to provide a clear and consistent understanding of the specification and claims, including the scope to be given such terms, the following definitions are provided.

***Alpha-fetoprotein-like immunoreactivity:*:** Any immune reactions caused by alpha-fetoprotein. Alpha-fetoprotein can be full-length or truncated, including isomers and splice variants of alpha-fetoprotein.

***Committed progenitors:*** Immature cells that have a single fate such as hepatocytic committed progenitors (giving rise to hepatocytes)) or biliary committed progenitors (giving rise to bile ducts). The commitment process is not understood on a molecular level. Rather, it is recognized to have occurred only empirically when the fates of cells have narrowed from that of a predecessor.

***Hepatic cells:*** A subpopulation of liver cells which includes hepatocytes and biliary cells.

***Liver cells:*** As used herein, the term "liver cells" refers to all type of cells present in normal liver, regardless of their origin or fate.

***Stem cells:*** As used herein, the term "stem cells" refers to immature cells that can give rise to daughter cells with more than one fate, that is they are pluripotent. Totipotent stem cells, such as embryonic stem cells (ES cells) or embryonic cells up to the 8 cell stage of a mammalian embryo, have self-renewal (self-maintaining) capacity in which the stem cell produces a daughter cell identical to itself. By contrast, determined stem cells, such as hemopoietic, neuronal, skin or hepatic stem cells, are pluripotent and have extensive growth capacity but have questionable self-renewal capacity. In the case of totipotent stem cells, some daughter cells are identical to the parent, and some "commit" to specific fate (s) restricting their genetic potential to that which is less than the parent's. In the case of determined stem cells, some daughter cells retain pluripotency and some lose it, committing to a single, specific fate.

***Hepatic progenitors:*** These cells give rise to hepatocytes and biliary cells. The hepatic progenitors include three subpopulations: "hepatic stem cells", "committed hepatocytic progenitors", and committed biliary progenitors, the last two being immature cells that are descendants of the hepatic stem cell and that have a single fate, either hepatocytes or biliary cells, but not both.

***Hepatic stem cells:*** A subpopulation of hepatic progenitors.

***Liver progenitors:*** A cell population from liver, including hepatic progenitors, hemopoietic progenitors and mesenchymal progenitors.

***Hemopoiesis:*** yielding blood cells with cell fates of lymphocytes (B and T), platelets, macrophages, neutrophils, and granulocytes.

***Mesengenesis:*** yielding mesenchymal derivatives with cell fates of endothelia, fat cells, stromal cells, cartilage, and even bone (the last two occurring in the liver only under disease conditions).

***Cell Therapy:*** As used herein, the term "cell therapy" refers to the *in vivo* or *ex vivo* transfer of defined cell populations used as an autologous or allogenic material and transplanted to, or in the vicinity of, a specific target cells of a patient. Cells may be transplanted in any suitable media, carrier or diluents, or any type of drug delivery systems including, microcarriers, beads, microsomes, microspheres, vesicles and so on.

***Gene Therapy:*** As used herein, the term "gene therapy" refers to the *in vivo* or *ex vivo* transfer of defined genetic material to specific target cells of a patient, thereby altering the genotype and, in most situations, altering the phenotype of those target cells for the ultimate purpose of preventing or altering a particular disease state. This can include modifying the target cell *ex vivo* and introducing the cells into the patient. Alternatively, a vector can be targeted to liver progenitor cells *in vivo* to deliver the exogenous genetic material and transfect the progenitors. Furthermore, genetically engineered progenitor cells can be used in a bioreactor as a therapy for patients or as source of biological products. As this definition states, the underlying premise is that these therapeutic genetic procedures are designed to ultimately prevent, treat, or alter an overt or covert pathological condition. In most situations, the ultimate therapeutic goal of gene therapy procedures is to alter the phenotype of specific target cell population.

***CD*:** "Cluster of differentiation" or "common determinant" as used herein refers to cell surface molecules recognized by monoclonal antibodies. Expression of some CDs are specific for cells of a particular lineage or maturational pathway, and the expression of others varies according to the state of activation, position, or differentiation of the same cells.

When the terms "one", "a," or "an" are used in this disclosure, they mean "at least one" or "one or more," unless otherwise indicated.

### II. Alpha-fetoprotein and albumin as diagnostic markers for hepatic lineages.

Alpha-fetoprotein (AFP) and albumin, both cytoplasmic proteins, are especially reliable markers for hepatic lineages. The expression of these proteins is the foundation for identification of the hepatic subpopulations from other cell types in the liver.

Human leukemia cell lines and normal T lymphocytes after *in vitro* stimulation can also express AFP. The data, however, do not address whether the AFP mRNA's in the leukemia cell lines and activated T lymphocytes are an identical form to the authentic AFP mRNA in hepatic cells. It has to be determined whether or not the expression of AFP or albumin mRNA's can be measured by routine protein assays, such as immunofluorescence, western blots, etc., because RT-PCR is the most sensitive technique known for identifying particular RNA templates.

Prior to the studies described herein, no one had ever investigated in detail the forms of AFP or albumin mRNA's in hemopoietic cells in human. Herein it is demonstrated that the expression of the variant forms of AFP and albumin in hematopoietic cells.

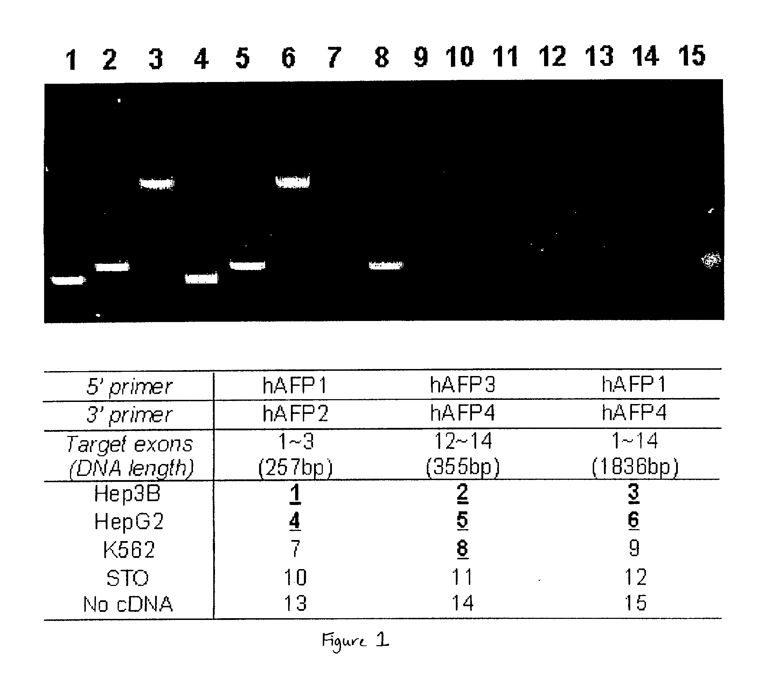

**Figure 1** illustrates the analysis of liver and non-liver cells by polymerase chain reaction (PCR) with primers to several exons of alpha-fetoprotein mRNA. PCR Analysis reveals truncated AFP in hemopoietic cells. RT-PCT using the primer combination of hAFP1, hAFP2, hAFP3, and hAFP4 was performed. M=molecular weight markers, lanes 1-3=Hep3B; lanes 10-12=STO fibroblasts; lanes 13-15=no RNA. Note, there is a shared band, a truncated AFP isoform, in lanes 2, 4, and 8. T here is a variant AFP isoform unique to liver cells noted in lanes 1 and 4. The complete AFP species is observed in lanes 3 and 6. The inventors have designed nine PCR primers in order to characterize variant forms of hAFP mRNA, as exemplified in Example 1. The coding sequence of AFP extends from exon 1 to exon 14. All primer combinations other than the one for exon 1 of AFP mRNA amplify the portion of the AFP mRNA in a human erythroleukemia cell line, K562, whereas all combinations detected AFP mRNA in human hepatic cell lines HepG2 and Hep3B. This demonstrates that variant forms of AFP mRNA contain from exon 2 to exon 14, as expressed in K562, but do not cover the entire coding sequence of AFP. The result suggests that the only useful primers for identifying hepatic cells are those that detect the portion of exon 1 of AFP, the expression of which is more provably restricted in a tissue-specific manner. The fact that exon 1 is unique to hepatic progenitor subpopulations enables one to use it as a probe for identifying hepatic versus hemopoietic progenitor cell types.

Since a truncated form of AFP is found in some subpopulations of hemopoietic cells, albumin is also analyzed in both hepatic and hemopoietic cells. Primers for albumin are developed in a fashion analogous to that for AFP (see above) and used to assess albumin expression in hepatic versus hemopoietic cell lines. As for AFP, a truncated form is found in K562, the hemopoietic cell line, and a transcript that is detected by the primer for exon 12-14.

This knowledge can be used to design and prepare specific primers of RT-PCR to determine the expression pattern of variant forms of AFP and albumin mRNA in hepatic versus hemopoietic cell populations. Herein, it is demonstrated that variant forms of both AFP and albumin mRNA can be found in hemopoietic progenitors. It means that when such sensitive assays are used, additional criteria, such as the use of an exon 1 probe for AFP, must be used to define hepatic from hemopoietic cell populations.

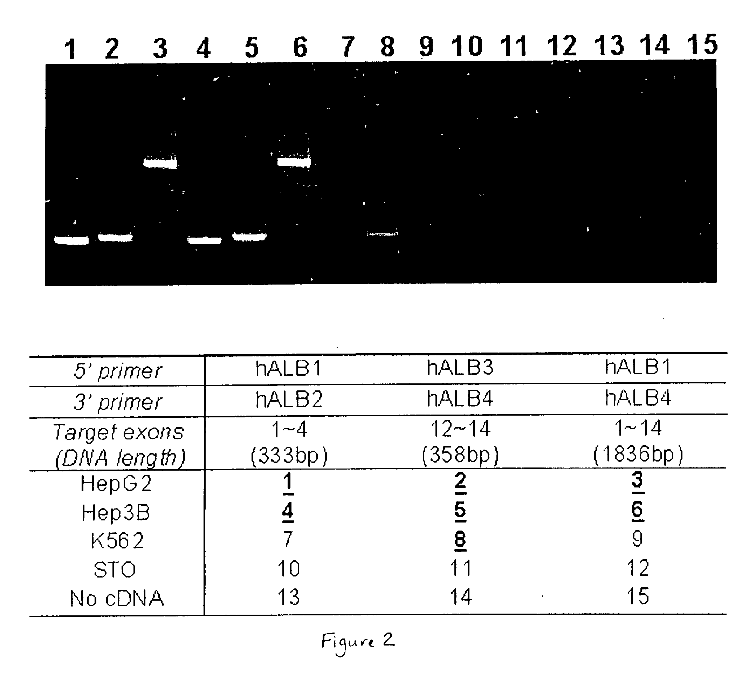

**Figure 2** illustrates the analysis of liver and non-liver cells by PCR to several exons of albumin. Since a truncated form of AFP mRNA is found in some subpopulations of hemopoietic cells, albumin is also analyzed in both hepatic and hemopoietic cells. Primers for albumin are developed in a fashion analogous to that for AFP (see above) and used to assess albumin expression in hepatic versus hemopoietic cell lines. As for AFP, a truncated form is found in K562, the hemopoietic cell line, and a transcript is detected by the primer for exon 12- 14.

Developmental studies of liver demonstrate that fetal liver is both a hepatopoietic and hematopoietic organ during intrauterine development. During various stages of liver development, the fetal liver contains large numbers of hematopoietic cells, especially of the erythroid lineage. Furthermore, there is an increasing awareness that hepatopoietic and hematopoietic systems are closely inter-related and the possibility exists that this interrelationship includes the joint expression of AFP and albumin, or perhaps isotypes of this protein. The fact that exon 1 of AFP is unique to hepatic progenitor subpopulations enables one to identify specific subpopulations of liver progenitor cells.

Although the PCR analyses reveal that hemopoietic progenitors can express both AFP and albumin mRNA species, the mRNA expression levels are very small. Indeed, when AFP and albumin are measured by flow cytometric analysis, no detectable AFP or albumin could be found in K562. Although both AFP and albumin are critical guides in the identification of hepatic cells, AFP is especially diagnostic of the hepatic progenitor cells after their purification by flow cytometry because of its intense expression in the hepatic progenitors. AFP is adopted also to estimate the purity of hepatic progenitors after any kind of fractionation strategy.

### III. Processing of Human Liver Progenitors

The inventors have established methods that optimally yield dissociated human liver progenitors from fetal or adult livers. The isolation of mature liver cells usually involves enzymatic and mechanical dissociation of the tissue into single cell suspensions followed by fractionation with density gradient centrifugation, centrifugal elutriation, differential enzymatic digestion protocols (i. e. hepatic stellate cells), and/or with selection using cell culture (reviewed in Freshney, "Culture of Animal Cells, A Manual of Basic Technique" 1983, Alan R Liss, Inc. NY). Density gradient centrifugation is used routinely by most investigators to eliminate what they assume to be debris and dead cells by discarding all fractions and retaining only the final pellet.

Whereas all other investigators use the final pellet after density gradient fractionation, the protocol disclosed herein is unique in that it makes use of the upper fractions of a density gradient and excludes the pellet. The novel variation to the density gradient centrifugation, as disclosed herein, is that the pellet is discarded and cells with a lower buoyant density (i. e., cells collecting at or near the top of the gradient) are retained. The inventors have found that younger (i. e. diploid) and cells more robust upon cryopreservation are present at the top of or within the Percoll density gradient, rather than in the pellet.

### IV. Debulking

Debulking is a process for enrichment of liver progenitors. The progenitors may be any of several lineages, including hepatic, hemopoietic, and mesenchymal. As the liver has a variety of mature cells, which can be tetraploid or polyploid, it is useful to remove some, or all, mature cells to prepare an enriched population of progenitors. It is advantageous but not essential to carry out the debulking step at 4 °C.

After preparation of a single cell suspension of liver cells, the cells are separated into multiple fractions according to cell size, buoyant density, or a combination of both. According to the invention the liver progenitor cells are less than 15 microns in diameter. Any separation method that separates such small cells from larger cells and from cell debris is suitable, including sedimentation velocity in culture medium (which can be basal medium or enriched medium), gradient sedimentation, chromatography using large pore size separation beads, among others. The gradient material can be polyvinylpyrrolidone-coated silica (Percoll), cross-linked sucrose (Ficoll), dextran or any known to those in the art, and prepared to be isotonic to prevent cell lysis, in, for example, phosphate-buffer saline or Eagle's basal medium (BME). The suspension of dissociated cells is typically applied to the top of a layer of the gradient material and subjected to a centrifugal field, while kept at 4 °C. Alternatively, the cell suspension may be applied to an apheresis unit, such as is used for isolation of blood components, i.e. plasmapheresis. Large cells, including mature parenchymal cells and tetraploid cells are sedimented faster than the small progenitors and diploid cells, and are removed. The design of the centrifugation protocol takes account of the sensitivity of cells to low oxygen tensions and minimizes the time for cell enrichment. The cell suspension can be enriched for hepatic progenitors by these methods. Furthermore, the debulking step can comprise centrifugal elutriation, panning based on cell surface adherence proteins, affinity chromatography or batch processing, tagging with fluorescent labels, zonal centrifugation, continuous-flow centrifugation, magnetic sorting after incubation with magnetic beads, *e.g*. magnetic beads complexed to antibodies, or combinations of these methods. The density gradient centrifugation can be a discontinuous gradient or a continuous gradient. The Percoll fraction is suitable for immediate use, cryopreservation, establishment in culture, or further enrichment. Further enrichment can be accomplished by panning, affinity selection, FACS sorting or any of the techniques known in the art and described above. Negative selection is accomplished by removal of cells expressing markers for CD45, glycophorin A, or other markers as mentioned below. Positive selection is accomplished by selection of cells expressing CD14, CD34, CD 38 or other markers indicative of expression of full-length alpha-fetoprotein, albumin, or both.

In another embodiment of debulking, non-progenitors are selectively removed by selective lysis. Red cells are lysed by brief exposure of the cell suspension to an isotonic solution of ammonium chloride, followed by dilution with culture medium and centrifugation to remove red cell "ghosts" and free hemoglobin. Similarly, non-progenitors are selectively and hydrolytically lysed by freezing using the cryopreservation mixture described below. The various methods of debulking remove polyploid cells, cells that express markers associated with mature hemopoietic cells, cells that express markers associated with mature hepatic cells, cells that express markers associated with mature mesenchymal cells, and combinations of these cells.

### V. Cryopreservation of human liver progenitors and their progeny

The cryopreservation methodologies disclosed herein are unique and distinct from the methods used in the prior art. Major distinctions are the use of different buffers and cryopreservation of a hepatic progenitor population which is low in density and, thus, buoyant in gradient centrifugation. The hepatic progenitors are small in size and diploid.

Successful cryopreservation of mature human liver cells is highly desired but has never been achieved in the art. Generally, successful cryopreservation is defined as the ability to freeze the cells at liquid nitrogen temperatures (-160-180°C) and then to thaw them, observe viabilities of >75% and with the ability to attach onto culture dishes. Using older methods, mature hepatocytes of rodent or human origin have viabilities of 30-40% with no ability to attach after freezing under the above conditions (for example see Toledo-Pereya, et al., U.S. Patent No. 4,242, 883; Fahy et al., U.S. Patent No. 5,217,860; Mullon et al., U. S. Patent No. 5, 795, 711; and Fahy et al., U. S. Patent NO. 5, 472, 876). These patents disclose a very poor viability (<50%) of cells, are dealing mainly with cell cultures (not individual cells in cell suspension) and require a prolonged exposure of the cells to the buffer prior to freezing.

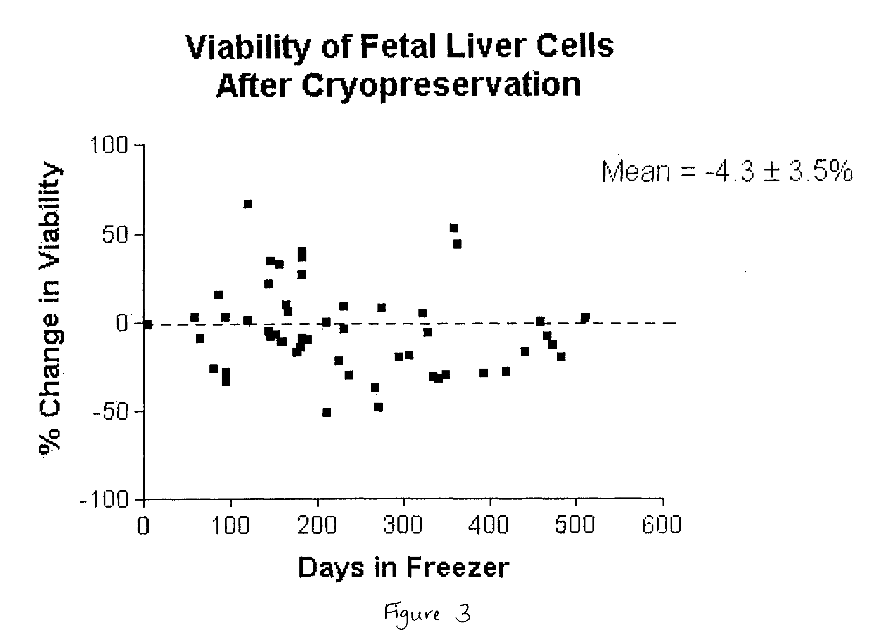

Figure 3 illustrates the excellent viability of liver cells which are cryogenically stored as disclosed herein. Data are expressed as the percent change in viability measured at the time of processing versus the time of thawing. These data indicate that the cryopreservation did not affect significantly the viability of the cells. There was no significant change in viability over a period extending to 550 days in storage. The special cryopreservation methodology disclosed herein includes the use of a novel buffer, a novel cell population, and optionally embedding the cells in forms of extracellular matrix. This methodology for the first time achieves a viability upon thawing that is not different from the viability measured prior to freezing, immediately after cell dispersion. Actual viabilities are variable due to the condition of the tissue upon arrival and the effects of preparation of the cell suspension using enzymatic and mechanical dissociation, and, in the present studies, averaged 77% for the fetal liver cells. The cryopreservation methodologies resulted in no significant loss in viability by the freezing process and in cells that could attach and expand *ex vivo* after thawing.

### VI. Immunoselection of Human Liver Progenitors

The invention teaches a method of isolating progenitors from human liver comprising providing a substantially single cell suspension of human liver tissue, and subjection the suspension to a positive or negative immunoselection. The method of immunoselection can comprise selecting from the suspension those cells, which themselves, their progeny, or more mature forms thereof express at least one marker associated with at least one of the cell lineages. These cell lineages can be hemopoietic, mesenchymal, hepatic, or some combination of these cell lineages. The cell selection step can include removing cells that express glycophorin A, CD45, an adult-liver-cell-specific marker, or combinations of these. Moreover, the selection method can include removing polyploid cells, cells that express markers associated with mature hemopoietic cells, cells that express markers associated with mature hepatic cells, cells that express markers associated with mature mesenchymal cells, or combinations thereof. The selection of cells can comprise selecting cells that express CD14, CD34, CD38, or combinations thereof. Furthermore, the method can identify and select mature hemopoietic cells that express glycophorin A, CD45, or a combination of these.

The immunoselection method is carried out in conjunction with debulking based on cell size, buoyant density, or a combination thereof. The selection method can select cells that express at least one marker associated with at least one cell lineage, which may be hemopoietic, hepatic, or mesenchymal. The selection of cells, their progeny, or more mature forms thereof can express at least one marker associated with at least one hepatic cell lineage. That lineage can be parechymal cells or hepatocytes, or biliary cells. Thu, the markers expressed by the cells can be CD14, CD34, CD38, or combinations thereof.

### VI. Cell Markers and Flow Cytometry

Using our current definition of liver progenitors as immature cell populations that express alpha-fetoprotein with or without expression of albumin, we have assessed markers that will select specifically for these cells using immunoselection technologies. A startling discovery has been that many of the markers (*i*.*e*. CD34) that are classical ones for hemopoietic progenitors, also identify hepatic progenitor subpopulations. Thus, single color sorts for CD34 resulted in significant enrichment (at least 9-fold) for cells that express AFP. However, not all of these AFP-positive cells can be verified to be hepatic progenitors. Based on the percentage that are albumin positive, we estimate that 80-90% of the cells are hepatic progenitors, and the others are either hepatic progenitors too immature to yet express albumin or possibly hemopoietic subpopulations that express alpha-fetoprotein.

This invention uses a unique flow cytometric sorting strategy. Using the combination of AFP and albumin expression as two uniquely defining features of hepatic progenitors, we have identified antigenic markers and other flow cytometric parameters that define the hepatic progenitor cells. The sorting strategies to date involve sorts for small cells (< 15, µ by measures of forward scatter), that are diploid (using fluorescence from Hoechst dye 33342), are agranular by side scatter, are negative for certain hemopoietic antigens (i. e. glycophorin A, the red blood cell antigen and CD45) followed by positive markers shared between hepatic cell subpopulations and hemopoietic cell subpopulations (*i.e.* CD14 and/or CD38). In the experiments described herein, the inventors identify hepatic progenitor cells by sorting for those cells that strongly express alpha-fetoprotein, weakly express albumin, and express CD14, CD34, CD38, or a combination thereof. Also, described herein is the evidence that hemopoietic cells also express AFP, albeit a truncated form. The inventors describe a novel cell population and process of isolation and identification of such a cell population. The success in the isolation and identification of the particular cell population of the invention is achieved partly through advanced methods of isolation, affinity debulking, high-speed fluorescence-activated cell sorting, greater speed and accuracy, and modified cryopreservation and culture techniques.

Applicants demonstrate flow cytometric sorting strategies and methods to purify liver progenitors from freshly isolated cell suspensions and/or from thawed cryopreserved liver cells. These methods involve 1) staining of the cells with several fluoroprobe-labeled antibodies to specific cell surface markers and 2) using a combination of negative and positive sorting strategies in multiparametric flow cytometric technologies. The methods for purification of specific lineage stages from human hepatic cell populations can be used with livers from any age donor, since the markers appear to be lineage-position specific.

The improved methods of labeling the cells, and a dramatically improved flow cytometer ("a MoFlo" flow cytometer from Cytomation which sorts cells at 40,000 cells/second and performs 8 color sorts) over that which was used in the past (Becton Dickenson's FACSTAR PLUS which sorts cells at 2000-6000 cells/second and performs 2-4 color sorts); assists in the successful isolation, and identification of this cell population.

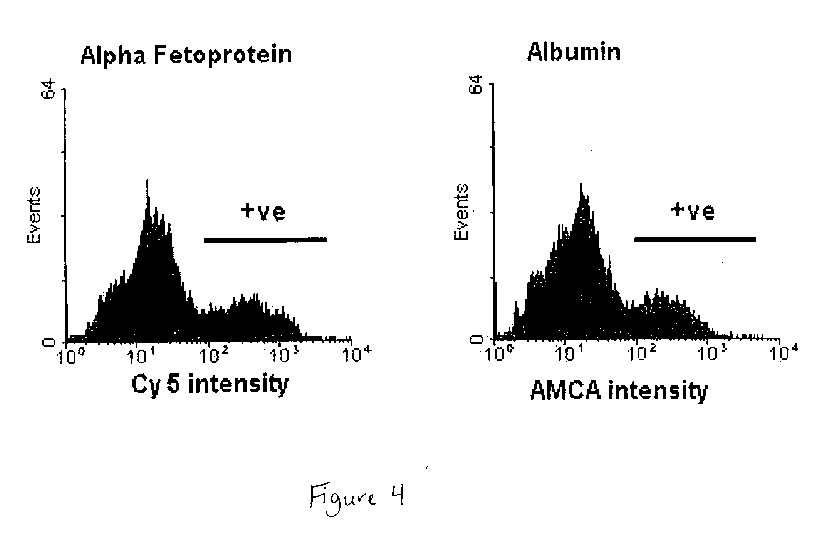

**Figure 4** illustrates a univariant FACS sort. The cell suspension is prepared for immunofluorescence analysis of alpha-fetoprotein (AFP) using antibodies conjugated to the red dye, Cy5, and for albumin using antibodies conjugated to the blue dye (AMCA). Thirty thousand cells are screened for red (AFP) and blue (albumin) fluorescence. The results show a clear group of cells positive for each protein. Further analysis shows that about 80% of the positive populations for each protein are represented by the same cells (*i*.*e*. co-expression of the two proteins). The expression of AFP and albumin like immunoreactivity is well defined in the cell suspensions, with a clear group of cells showing a clear differentiation from the background signal. Alpha-fetoprotein is expressed in 6.9±0.86% of cells in unfractionated cell suspensions and albumin is present in 7.7±1.1%. Among AFP positive cells 75.6±4.9% co-expressed albumin while 80±5.5 % of albumin positive cells also expressed AFP. Thus, approximately 25% of cells expressing alpha-fetoprotein do not express albumin and 20% of cells expressing albumin do not express alpha-fetoprotein.

The proportions of cells bearing the principle surface markers used in this work are shown for complete cell suspensions (i. e. including red cells) in Table 2 (GA= glycophorin A, a surface marker on red blood cells)

**Table 2 : Percentage of CD Positive Cells in Original liver cell Suspension and percentage of these that are positive for AFP**

| | **Cd14** | **CD34** | CD38 | CD45 | GA |
|---|---|---|---|---|---|
| Unfractionated | | | | | |
| % in population | 3.7±0.8(8) | 2.8 ±0.5 | 2.2 ±0.4 | 2.6 ±0.5 | 36.8±5 |
| %AFP postive | 81.7±2.2 | 72.6±4.6 | 57.6±4.6 | 22.2±4.4 | 2.3±0.6 |

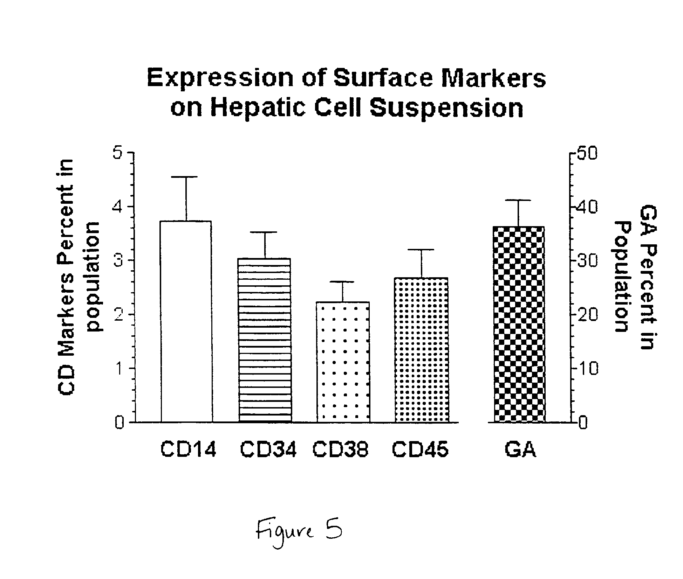

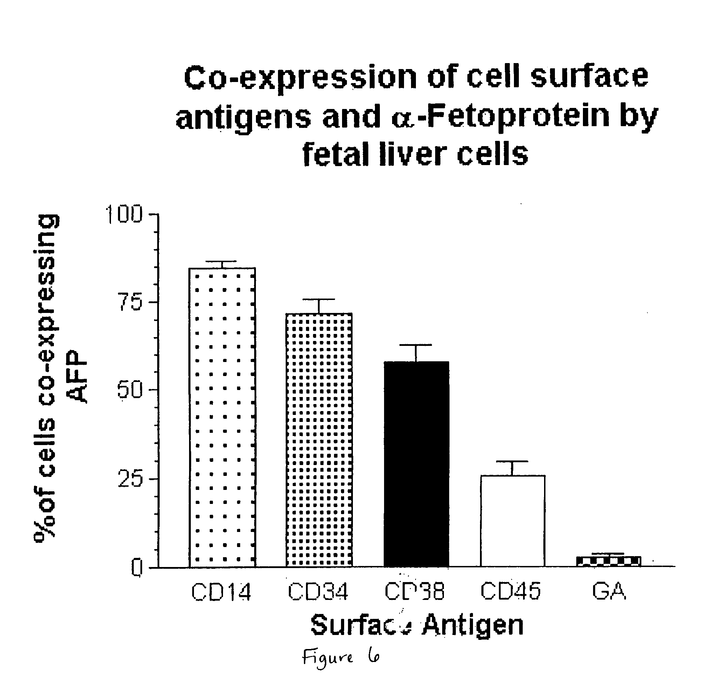

**Figure 5** illustrates the percent of cells expressing surface markers CD14, CD34, CD38, CD45, and Glycophorin A (GA) in unfractionated liver cell suspensions. Note that the GA data is plotted on the right axis to preserve scale. Figure 6 illustrates the percentage of cells in the original cell suspension expressing alpha-fetoprotein and other antigenic markers. Mean ± SEM for percent of cells positive for alpha-fetoprotein (AFP) and specific cell surface markers (CD14, 34, 38, 45 and glycophorin A). Clearly, glycophorin A (GA) positive cells (i.e. erythroid cells) represent a major component of the cell mass but an insignificant fraction of the AFP-positive cells.

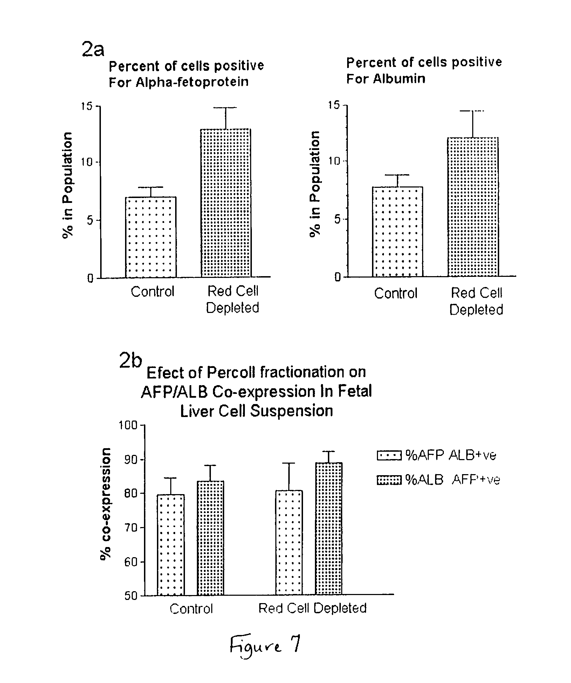

**Figure 7** (top) illustrates the co-expression of alpha-fetoprotein and albumin. The expression of alpha-fetoprotein (left panel) and albumin (right panel) in suspensions of fetal liver cells with or without selective depletion of red cells using Percoll fractionation. The percent of AFP positive cells co-expressing albumin is also increased to 80.5±8.2% and the proportion of albumin-positive cells co-expressing AFP increased to 89±3.1%, though neither change is statistically significant.

**Figure 7** (bottom) illustrates the effect of debulking by Percoll fractionation on alpha-fetoprotein and albumin co-expression. The proportion of cells expressing both alpha-fetoprotein and albumin, expressed as a percentage of AFP or albumin positive cells. Data for cells with and without red cell depletion are shown using Percoll fractionation. Thus, when cell suspensions are depleted of red cells by Percoll fractionation the proportion of cells expressing AFP is increased significantly to 12. 91. 9% and those expressing albumin to 12.1+2.3%.

The result of this procedure on the proportion of cells bearing the surface markers are shown in Table 3, together with the proportion of each subgroup showing positive staining for AFP.

**Table 3 : Percentage of CD Positive Populations in Liver Cell Suspension after Depletion of Red Cells and percentage of these that are positive for AFP**

| | CD14 | CD34 | CD38 | CD45 | GA |
|---|---|---|---|---|---|
| Red cell depleted | | | | | |
| % in population | 7.4±1.3 | 3.4±0.5 | 4.8±0.9 | 8.2±0.3 | 27.5±4.7 |
| % AFP postive | 89.8±1.3 | 77.1±2.9 | 53.5±1.3 | 32.5±1.3 | 1.8±0.9 |

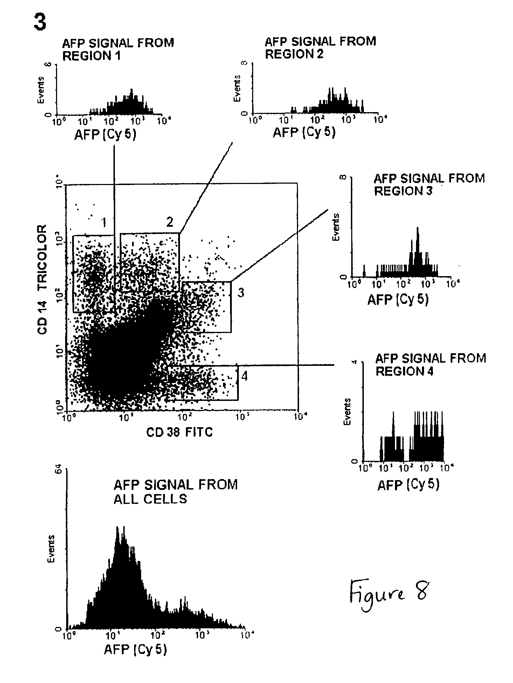

Figure 8 illustrates a FACS analysis of fetal liver cell suspension for co-expression of CD 14, CD38 and AFP. The bivariate scattergram shows the distribution of TriColor staining for CD14 (ordinate) versus FITC staining for CD38 (abscissa). Gates are created to select specific cell groupings according to the CD14 and CD38 signals. These are then used to display the intensity of AFP staining in each of these subgroups. The AFP results show that a high level of enrichment for AFP is produced by selecting cells positive for either CD38 or CD14. The AFP signal generated from the entire cell suspension (30, 000 cells) is shown at the lower left. In most cases, the presence of AFP in the subgroups selected by cell surface marker is distributed continuously with a clear preponderance of cells showing staining intensities in the positive range. However, the distribution of CD38 positive cells with respect to co-expression of AFP was unique. In CD38-positive cells a bimodal distribution for AFP co-expression is apparent in which two distinct groups of cells are apparent, one group positive for AFP, the other negative.

The results show that alpha-fetoprotein (AFP) is present in 7% of the cells in single cell suspensions of fetal liver tissue (i.e. in the original cell suspension). The antibody to glycophorin A (an antigen on red blood cells, erythrocytes) is found to identify a subpopulation of cells that do not express AFP. Thus, cells expressing this antigen (i.e. erythroid cells) are excluded from cells intended for characterization of hepatic progenitors. The CD38 antigen identifies a population of cells that shows significant enhancement in the proportion of AFP positive cells (i. e., greater than 7 times the proportion in unfractionated samples. Both antigens show a number of isoforms, depending on whether or not there are sections of the molecule, encoded by splicing variants, present. Antibodies are available that identify the various isoforms.

The classic marker for hemopoietic progenitor cells, CD34, is found to be present on many cells that also express AFP. The sorting of cells positive for CD34 results in enrichment of AFP-positive cells at least 9 fold over that found in the original cell suspension (67%, in the CD34-positive cells vs 7% in the original cell suspension). However, the most effective single antibody for enrichment of AFP positive cells is CD 14, which produces a greater than 11 fold increase in the proportion of these cells compared to the original population (81 % versus 7%).

It would seem that the yield of AFP positive cells could be improved by using a combination of surface markers. Thus, the extent of co-expression of AFP with selected combinations of surface markers is determined to establish the extent to which the selection the intracellular marker can be increased. The data are expressed as the proportion of AFP positive cells expressing surface markers (termed the "yield" of AFP positive cells) and as the proportion of all AFP positive cells that appear in the population defined by the surface marker (termed the "enrichment" factor for AFP positive cells). Results for combinations of CD 14, CD34 and CD38 are shown in **Table 4** together with the results from individual markers for comparison.

**Table 4.**

| | **CD14** | **CD34** | **CD38** | **CD14+CD38** | **CD14+CD34** |
|---|---|---|---|---|---|
| Enrichment | 80.6±2.6 | 66.7±4.7 | 53.8±4.5 | 66.9±3.5 | 68.2±3.9 |
| Yield | 39.8±2.6 | 26.9±4.4 | 22.0±2.7 | 50.6±2.7 | 52.0±5.5 |

### Enrichment. Percent of cells expressing either (or both) of the surface markers that are also positive for AFP.

### Yield. Percent of all AFP-positive cells that also expresses one or both of the surface maker combination.

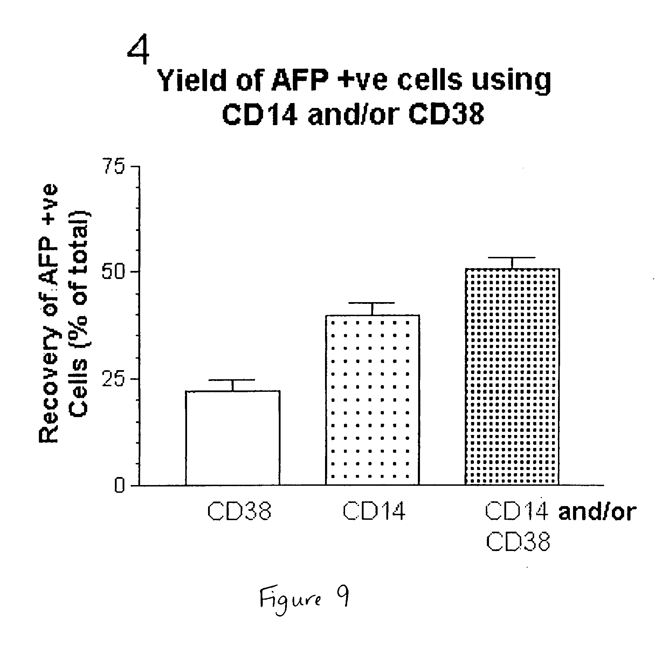

Figure 9 illustrates how selection for CD14 and CD38 enriches for AFP positive cells. The proportion of AFP-positive cells in cell suspensions prepared from fetal liver is enhanced dramatically by selecting cells with positive surface labeling for the markers CD38 and CD14.

The combination of the two markers produces a significantly better enrichment of AFP-containing cells than that obtained with either marker alone.

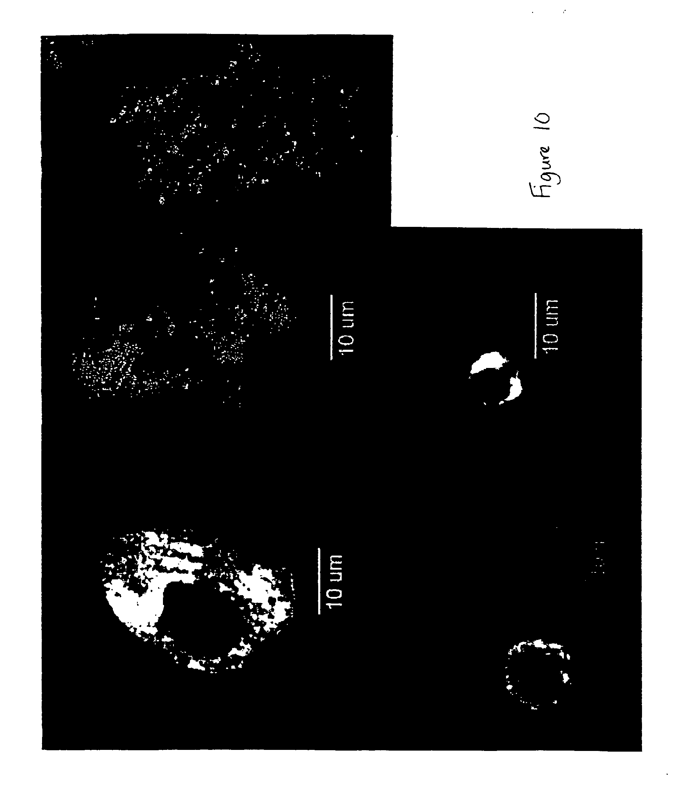

**Figure 10** illustrates fluorescence microscopy of human hepatic progenitor cells. Representative hepatic progenitor cells from the fetal liver stained for AFP content. Cell sizes indicate that both early progenitors and more advanced hepatic progenitors are present. The morphology of cells staining positive for AFP is variable and encompassed the entire range of cell size and shape in the cell suspension from fetal livers but not adult liver. The largest of the AFP-positive cells, approximately 12-15µ, is much smaller than mature hepatocytes, ranging in size from 20-50µ).

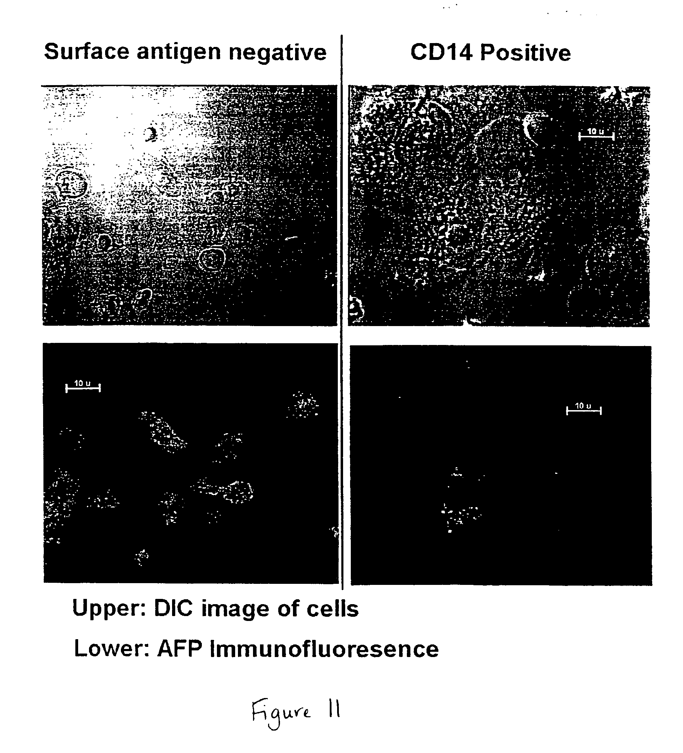

**Figure 11** illustrates representative cells selected by expression of AFP. The cells with positive staining for CD14 (right side) are characteristic of hepatoblasts. The cells with negative staining for surface markers are smaller and consistent in size and morphology with early hepatic progenitors. In all cases a certain proportion of AFP positive cells show no expression of any surface antibodies used in this study. The appearance of these AFP-positive "null" cells is illustrated in Figure 11 where they can be compared with the appearance CD14 positive/AFP positive cells sorted from the same suspension. It is clear that while both cell types are positive for AFP, the cells staining negative for surface antigens are consistently smaller and less complex than the CD 14 positive cells.

Thus, the probable markers for sorting hepatic progenitors are: Glycophorin A⁻, CD45⁻, and one or more CD14⁺, CD34⁺, CD38⁺, diploid, agranular (by side scatter), less than 15µ (by forward scatter). The phenotype of these sorted cells is small cells (< 15, µ), with little cytoplasm (high nucleus/cytoplasm ratio), albumin⁺ and/or AFP⁺.

### VII. Confocal Characterization of Alpha-Fetoprotein Expressing Cells in Fetal and Adult Human Liver.

Confocal microscopy has been used to obtain the images from human fetal and adult cells that express alpha-fetoprotein. This methodology enables one to observe the morphology and size of these cells and to show directly the location of intracellular proteins, such as AFP and ALB, and that of membrane surface markers such as CD34 and CD38.

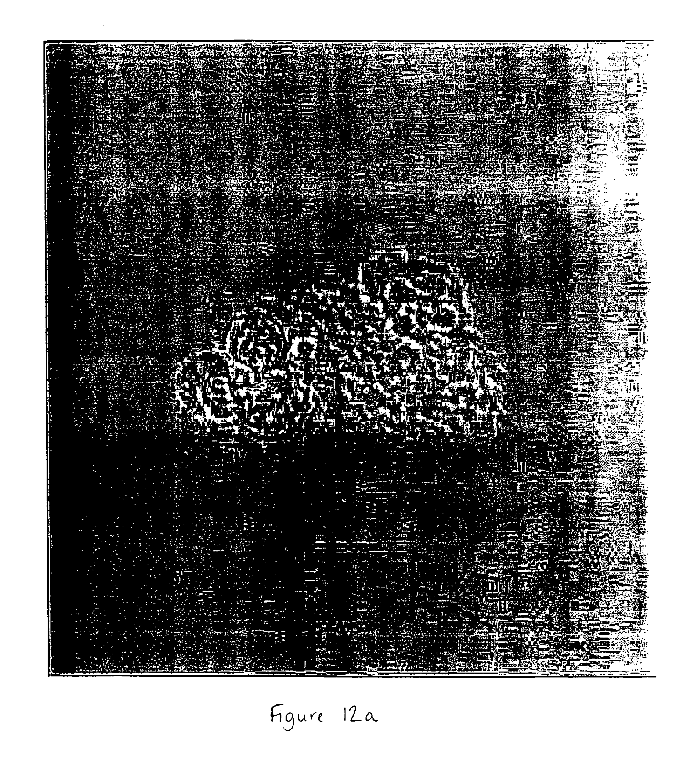

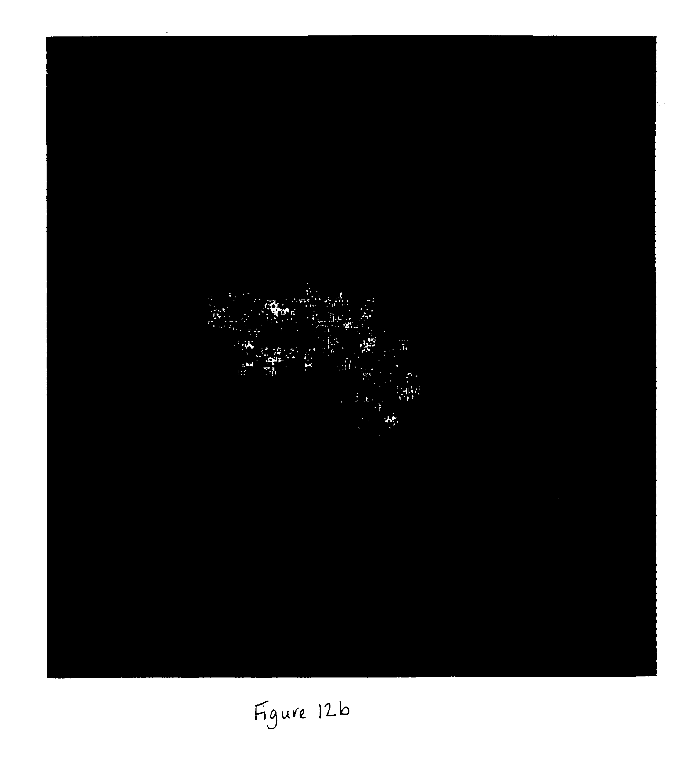

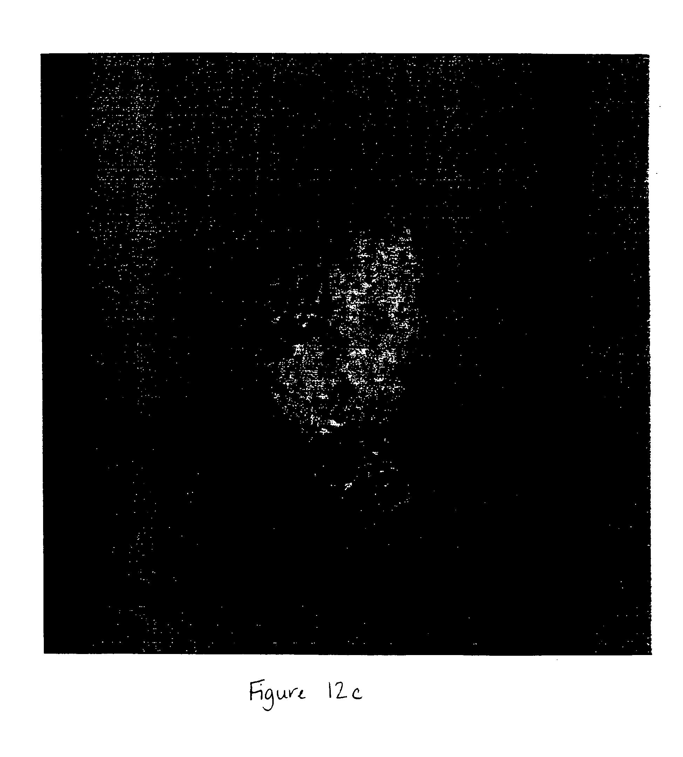

**Figure 12** illustrates confocal miscroscopy of alpha-fetoprotein expressing cells, that is, hepatic progenitors in adult human livers. The figure shows three view of one field, and that there are two AFP-positive cells in this field. The overlay of panel (A) and panel (B) is shown in panel (C) and indicates the morphology of AFP positive cells (colored pink, in the original) in a group of liver cells.

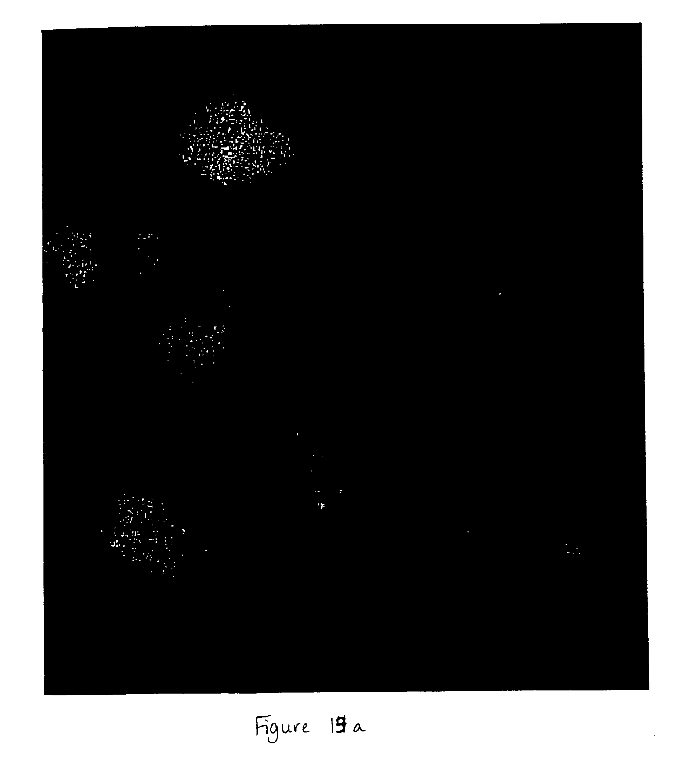

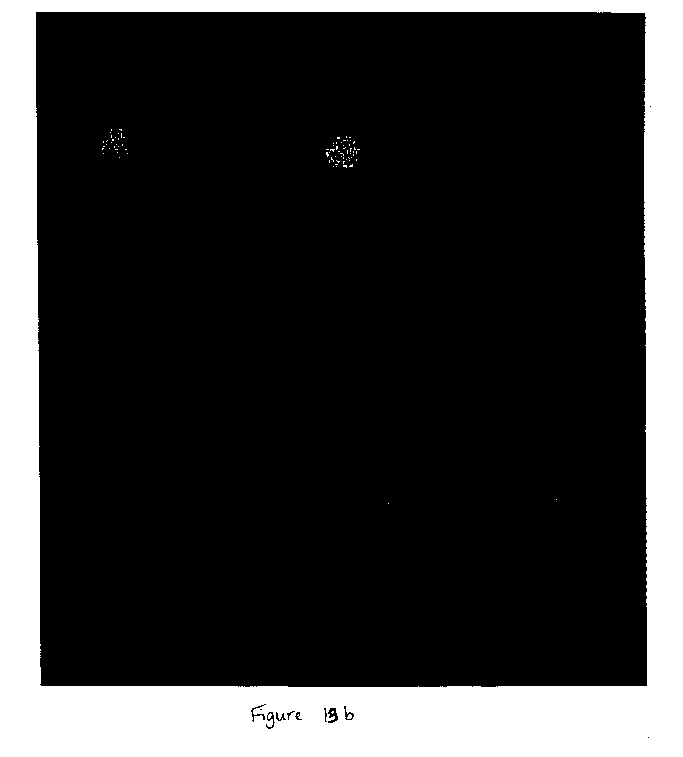

**Figure 13** illustrates cells that are labeled with calcein (A) to show all cell types. Fig. 13 (B) consist of the same cells co-expressing AFP and showing that only two cells are AFP-positive. Cell size is not a factor for AFP positivity.

AFP-expressing cells are found in both fetal and adult livers. Fetal livers, as expected, have the highest percentage (6-7%), whereas adult livers have a small percentage (<1%) and with the numbers declining with age of the donor. The few hepatic progenitors found in adult livers can be enriched significantly through the Percoll fractionation process to yield up to 2% of the cells in Percoll fractions 1 and 2 from the adult livers (Table 5). No AFP-expressing cells are found in a liver from donors older than 71 years of age.

**Table 5 shows the cell size and viability from Percoll-isolated fractions of adult liver cells. Smaller cells (fractions 1-3) have higher viability than larger cells (fraction 4) after being cryopreserved under the same cryopreservation condition.**

| **Fraction** | **Viability (%)** | **Cell Size (µm)** | % AFP+cells |
|---|---|---|---|
| Fraction 1 | 82 | >12 | 0.5-1% |
| Fraction 2 | 84 | 10-15 | 2 % |
| Fraction 3 | 85 | 15-25 | <02 % |
| Fraction 4 | 56 | 25-50 | <0.01% |

These results suggest that donor organs useful for liver cell therapies as well as for organ transplantation will consist of those from of young donors (up to about 45 years of age. Furthermore, the livers from geriatric patients (>65 years of age) will be inappropriate donors for cell therapies and perhaps also for whole organ transplants, especially for children, since they will have little if any regenerative capacity from hepatic progenitors and only the intermediate or minimal regenerative capacity known to be available from the mature cells.

### VIII. Maturational Lineage

Therefore, adult liver contains a hepatic progenitor cell population capable of growth and differentiation into hepatocytes and biliary cells under both normal and disease conditions. This invention stands for the proposition that every position in the liver lineage is a distinct maturational stage, and that there are multiple stem cell populations in the liver.

Surprisingly, the embryonic liver of the present invention yields progenitor cells for 3 separate maturational lineages : hepatopoiesis, with cell fates of hepatocytes and biliary cells (bile duct); hemopoiesis, with cell fates of lymphocytes (B and T), platelets, macrophages, neutrophils, and granulocytes; and mesengenesis, with cell fates of endothelia, fat cells, stromal cells, cartilage, and even bone (the last two occurring in the liver only under disease conditions).

In general, stem cells are immature cells that can give rise to daughter cells with more than one fate. The stem cells produce daughter cells, some of which are identical to the parent and some of which "commit" to a specific fate. The commitment process is not understood on a molecular level. Rather, it is recognized to have occurred only empirically when the fates of cells have narrowed from that of a predecessor. "Committed progenitors" are defined as immature cells that have a single fate such as hepatocytic committed progenitors (giving rise to hepatocytes) or biliary committed progenitors (giving rise to bile ducts).

The transitions from the stem cell to the adult cells occur in a step-wise process yielding a maturational lineage in which cell size, morphology, growth potential and gene expression is tied to the lineage. The metaphor of aging is useful in defining the process. The "young" cells have early gene expression and the greatest growth potential; the cells late in the lineage have "late" gene expression and usually are limited in their growth or do not grow at all. The late cells can be considered "old" or in biological terms, apoptotic, and ultimately are sloughed off. The maturational lineage process results in a natural turnover for the tissue and allows for regeneration after injuries. Tissues differ in the kinetics of the maturational process. The maturational lineage of the gut is quite rapid with a complete cycle occurring in less than a week; that of the liver is slow occurring, and in the rat liver is about a year.

The rat liver forms in embryonic life at about day 10, referred to "asembryonic day 10" or E10, with the invagination of the cardiac mesenchyme by endoderm located in the midgut region of the embryo (Zaret, K. 1998. Current Opinion in Genetics & Development. 8:526-31.). Earliest recognition of liver cells in the embryos has been by achieved using in situ hybridization studies for mRNA encoding alpha-fetoprotein (AFP) ( (Zaret, K. 1998. Current Opinion in Genetics & Development. 8:526-31; Zaret, K. 1999 Developmental Biology (Orlando). 209 : 1-10). AFP-expressing cells are observed in the midgut region of the embryo near the mesenchyme that produces the heart on day 9-10 in all rat and mouse livers assayed. The liver becomes macroscopically visible by E 12 and is about 1 mm in diameter by E 13.

In parallel, hemopoiesis occurs with the first identifiable hemopoietic cells appearing by E15-E16 (in rodents) and by the 3^{rd} to 4^{th} month (in humans) and with the peak of erythropoiesis (formation of erythroid cells or red blood cells) occurring by E18 (in rodents) and by the 5^{th}-6^{th} month (in humans). At the peak of erythropoiesis, the numbers of these red blood cells dominate the liver and account for more than 70% of the numbers of cells in the liver. The end of the gestational period is on day 21 in rodents and 9 months in humans. Within hours of birth, the numbers of hemopoietic cells decline dramatically such that by 2 days postnatal life (rodent) and within a week or two (human), the vast majority of the hemopoietic cells have disappeared having migrated to the bone marrow. No one knows the cause for the migration of the hemopoietic cells. There are however two dominant speculations.

First, the hemopoietic progenitors prefer relatively anaerobic conditions and flee to the bone marrow (which is relatively anaerobic) with the elevated oxygen levels in the liver with the activation of the lungs; and second, the loss of the pregnancy hormones are the cause of the migration. Postnatally, the loss of the hemopoietic progenitors in the liver is associated with a dramatic reduction in the numbers of hepatic progenitors and a parallel increase in the numbers and maturity of the hepatocytes and biliary cells. Full maturity of the liver is completed by 2-3 weeks postnatal life (in rodents) and within a few months (humans). By then the remaining hepatic progenitor cells are localized to the regions of the portal triads in the periphery of each liver acinus.

Thereafter, the classic architecture of the liver acinus is established with each acinus being defined peripherally by six sets of portal triads, each one having a bile duct, an hepatic artery and an hepatic vein, and in the center a central vein that connects to the vena cava. Plates of liver cells, like spokes in a wheel, extend from the periphery to the center. By convention, the plates are divided into three zones: Zone 1 is near the portal triads; zone 2 is midacinar; and zone 3 is near the central veins. The only diploid cells of the liver are in zone 1; tetraploid cells are in zone 2; and tetraploid, octaploid and multinucleated cells are in zone 3. The pattern is highly suggestive of a maturational lineage that ends in an apoptotic process ((Sigal, S. H., S. et al. 1995. Differentiation. 59 : 35-42.).

### IX. Implications of Lineage Concept in Pre-clinical and Clinical Studies of Liver Biology.

The *in vitro* and *in vivo* growth and differentiation characteristics of the cell population disclosed herein are in agreement with the concept and implications of a lineage-position lineage model in liver. For example, in an in vitro parenchymal culture, the ability of the parenchymal cells to divide and the number of cell divisions are predicted to be strictly lineage-position dependent. Therefore, periportal parenchymal cells should have greater division potential than pericentral ones. This explains the long-standing mystery of why primary cultures of liver, the most renowned regenerative organ in the body, show such limited cell division in culture.

Stem cells and their transformed counterparts, hepatomas, are predicted to express early genes such as alpha-fetoprotein and insulin-like growth factor II, but not genes expressed later in the lineage. In the maturity-lineage model no hepatoma should express late genes, because full progression through the lineage requires undisturbed regulation of differentiation, growth, and cell cycling. This indeed has been observed in the cell population disclosed herein. Molecular biological studies comparing liver-specific gene expression in embryonic versus adult tissues define several classes of genes: those diagnostic of the compartments (stem cell, amplification, differentiation); those expressed zonally and potentially crossing compartmental boundaries; and those expressed early, middle, or late in the lineage but discretely in one few cells. Various morphological and gene expression patterns of primary liver tumors may be understood by studying the cell population disclosed herein. If tumors represent the proliferation of transformed stem cells with varying capacities of differentiation, the common expression of alpha-fetoprotein in hepatomas is not an induced tumor marker but an indicator of an expanded immature cell population that normally expresses alpha-fetoprotein.

The isolated cell population disclosed herein has a great impact on the success of liver-directed cell and/or gene therapy. The Examples identify key conditions in which nonhuman primate and human hepatic progenitors can be successfully cryopreserved.

Because of the ability to significantly expand in vitro, the cell population disclosed herein , similar to cells in hemopoietic lineage, can be used as a "punch biopsy material" to provide the cell seed for *ex vivo* expansion. This would eliminate the necessity for major invasive surgical resection of the patient's liver.

Once the human hepatic progenitors are established in culture, gene transfer is performed. This can be accomplished with a number of different gene delivery vector systems. An important consideration at this point is that successful gene transfer requires a rapidly growing culture, and since human hepatic progenitors of the invention significantly divide under normal physiological conditions, these cells are ideal candidates for gene transfer to liver. Also, the growing characteristics of the cell population disclosed herein permits the use in an *ex vivo* gene transfer using certain gene delivery vectors (i. e., retroviral vectors) which will require cell proliferation for efficient gene insertion and expression.

An alternative approach for gene therapy is to design vectors that target the progenitors specifically and then to inject the vector, coupled with the gene of interest, directly into the patient. The vectors would target and modify the endogenous progenitor cell population.

The progenitor cell population disclosed herein can be used in an autologous or allogeneic liver-directed cell or gene therapy. Clearly, the use of autologous hepatic progenitors will eliminate a significant concern regarding rejection of the transplanted cells. The cell population disclosed herein is particularly attractive for allogenic cell transfer, because their antigenic profile suggests minimal immunological rejection phenomena. Moreover, other cellular elements, such as blood cells, endothelial cells, Kupffer cells, that are known to be highly immunogenic are substantially eliminated through the purification process.

Once the autologous or allogenic hepatic progenitors are isolated purified and cultured, they can be genetically modified or remain intact, expanded *in vitro,* and then transplanted back into the host. If genetic modification is desired, after genetic modification and before transplant, those genetically modified cells may be expanded and/or selected based on the incorporation and expression of a dominate selectable marker. Transplant can be back into the hepatic compartment or an ectopic or heterotopic site. For transplant into the hepatic compartment, portal vein infusion or intrasplenic injection could be used. Intrasplenic injection may be the administration route of choice because hepatic progenitors transplanted via an intrasplenic injection move into the hepatic compartment.

Additional medical procedures may assist in the efficacy of hepatic engraftment of the transplanted hepatic progenitors. Animal models have demonstrated that in partial hepatectomy, administration of angiogenesis factors, and other growth factors aide in the engraftment and viability of the transplanted hepatocytes. An alternative approach is to transplant the genetically modified hepatocytes to an ectopic site.

To date, the cell therapy approaches with respect to liver have shown little efficacy. This maybe due to the fact that the donor cells being used are predominantly adult liver cells and are short-lived after isolation and reinjection. In addition, the use of adult cells results in strong immunological rejection. The hepatic progenitor cells disclosed herein offer greater efficacy because of their limited capacity to elicit immunological rejection phenomena and because of their extensive regenerative potential.

With respect to gene therapy, the ongoing efforts make use of "targeted injectable vectors," the most popular route for clinical therapies under development. These approaches have had limited efficacy due both to immunological problems and transient expression of the vectors. The only routes for gene therapy that have proven merit-worthy have been *ex vivo* gene therapy and have been done almost exclusively in hemopoietic progenitor cells. We predict that *ex vivo* gene therapy with progenitor cells (or use of injectable vectors somehow targeted to those progenitor cell populations) will prove more effective, since the vectors can be introduced *ex vivo* into purified subpopulations of the progenitor cells; the modified cells selected and reintroduced *in vivo.* The advantages of the progenitor cells are their enormous expansion potential, their minimal, if any, induction of immunological reactions, and their ability to differentiate to produce the entire lineage of mature cells.

### X. Common or Interdependent Lineages

The improved methodologies enabled the inventors to more closely study and characterize hepatic progenitors. These studies revealed a specially close relationship between hepatic progenitors and hemopoietic progenitors suggesting a close relationship between these two lineages. Indeed, these studies show that the progenitor cells of the hepatic and hemopoietic lineages share numerous antigenic markers (CD14, CD34, CD38, ), share biochemical properties (i. e. transferrins, glutathione-S-transferases, and a truncated isoform of alpha-fetoprotein), and have extensive overlap in the culture requirements (forms of extracellular matrix and specific hormonal requirements) for expansion *ex vivo.* The progenitor cells of both lineages are located in the same sites within the liver acinus. Finally, paracrine signaling is present throughout the cells of the two maturational lineages; that is signals produced by each of the lineages regulates cells in the other lineage. Indeed, it may be concluded that there may be a common lineage or at the very least interdependent lineages between the hepatic and hemopoietic cells.

The cell populations disclosed herein are purified and utilized to yield either myelo-hemopoietic cells or hepatic derivatives depending on the conditions under which the cells are isolated and cultured. Therefore, bioreactor systems inoculated with cell populations sorted for a set of antigens that defines both hepatic and hemopoietic progenitors (*e.g*. CD38+, CD45+) can result in cell populations with multiple fates. The fate depends on how the cells are reintroduced *in vivo* or under what culture conditions the cells are placed. Another important aspect of the cell population disclosed herein is that they display a specific hemopoietic stem cell surface antigen CD34. CD34 positive cells of bone marrow has been used as a convenient positive selection marker for hemopoietic stem cells. However, there are increasing number of reports which cast doubt on the specificity of CD34 antigenic marker for hemopoietic stem cells (Nakauchi H. Nature Medicine 4: 1009-1010 (1998)). Experimental evidence demonstrates the existence of cells in the CD34 negative population of human bone marrow and cord blood that can repopulate the bone marrow of immunodeficient mice.

This invention, as disclosed herein, discloses ways to purify both the hemopoietic and the hepatic progenitor cell populations which can be used subsequently in the clinical and pre- clinical programs, utilizing the close relationships between the hepatic and hemopoietic cells.

The uses for human hepatic progenitors are many and diverse. They include : 1) research on human cells; 2) production of vaccines or antivirals; 3) toxicological studies; 4) drug development; 5) protein manufacturing (using the cells as hosts for production of various human-specific factors); 6) liver cell therapies; 7) liver gene therapies; 8) bioartificial livers that can be used in research, toxicological and antimicrobial studies, protein manufacturing, or clinically as a liver assist system. Considering the possibility of a common lineage between hemopoiesis and hepatopoiesis, as advanced by the inventors of this invention, the same cells can be used both for hepatic or hemopoietic fates depending upon the microenvironment in which they are placed.

The availability of highly purified human hepatic progenitor cells will enable much more extensive research on human cells, will facilitate the development of successful forms of liver cell and gene therapy, and should enable the development of human bioartificial livers for use both in research and as clinical assist devices. At present, the limited supply of healthy human tissues precludes clinical programs in liver cell therapy or in human bioartificial livers.

The progenitor cell populations should have sufficient expansion potential to overcome, or at least greatly alleviate, that limited supply.

### EXAMPLES

### Example 1

Analysis of variant forms of AFP and albumin expressed in hepatic versus other cell types.

*Cell lines*: Two human hepatomas, Hep3B and HepG2, are maintained in Eagle's MEM supplemented with 1 mM sodium pyruvate, 2mM L-glutamine, 50 U/ml penicillin, 50, ceg/ml streptomycin, 0. 1 mM MEM non-essential amino acid solution, 5 4gel insulin and 10% FBS. A human erythroleukemia cell line, K562 and a mouse embryonic fibroblast cell line, STO, are maintained in DMEM/F12 supplemented with 2 mM L-glutamine, 50 U/ml penicillin, 50, ag/ml streptomycin, 5 x 10-5M 2-ME and 10% FBS.

RT-PCR: Total RNAs are extracted from Hep3B, HepG2, and STO by the method of Chomcznski and Sacchi N. Anal. Biochem 162: 156-159 (1987). The cDNAs are synthesized by oligo-dT priming and subjected to PCR amplification using primer sets designed by the inventors and prepared for human AFP or albumin. The primer sequences are as follows

### For AFP

SEQ ID 1 haFP1: 5'ACCATGAAGTGGGTGGAATC-3',
SEQ ID 2 haFP2: 5'-CCTGAAGACTGTTCATCTCC-3',
SEQ ID 3 haFP3: 5'-TAAACCCTGGTGTTGGCCAG-3', °
SEQ ID 4 haFP4 5'-ATTTAAACTCCCAAAGCAGCAC-3',
: SEQ ID 5 hAFPexon2 : 5'-CTTCCATATTGGATTCTTACCAATG-3'.
SEQ ID 6 hAFPexon3 : 5'-GGCTACCATATTTTTTGCCCAG', SEQ ID 7 hAFPexon4 : 5'-CTACCTGCCTTTCTGGAAGAAC-3', SEQ ID 8 hAFPexon5 : 5'-GAGATAGCAAGAAGGCATCCC-3', and SEQ ID 9 hAFPexon6 : 5'-AAAGAATTAAGAGAAAGCAGCTTG-3', for albumin: SEQ ID 10 hALB 1 : 5'-GGCACAATGAAGTGGGTAACC-3', SEQ ID 11 hALB2 : 5'-CCATAGGTTTCACGAAGAGTTG-3', SEQ ID 12 hALB3 : 5'-GCCAGTAAGTGACAGAGTCAC-3', SEQ ID 13 hALB4 : 5'-TTATAAGCCTAAGGCAGCTTGAC-3', The combinations of the primers are as follows : For AFP : hAFP 1 and hAFP2, hAFP3 and hAFP4, hAFP 1 and hAFP4, hAFPexon2 and hAFP4, hAFPexon3 and hAFP4, hAFPexon4 and hAFP4, hAFPexon5 and hAFP4, and hAFPexon6 and hAFP4.
   For albumin : hALB 1 and hALB2, hALB3 and hALB4, hALB 1 and hALB4, PCR is performed in a total volume of 5041 consisting of IkiM each primer, 200, uM each dNTP, 50mM KCI, 1. 5mM MgCI2, 10mM Tris HCl, pH 8. 3, and 1. 25U Amplitaq polymerase (Cetus Corp). Samples are heated to 94°C for 3 min followed by amplification for 30 cycles of 2 min at 94°C, 2 min 62°C, and 3 min at 72°C. After the last cycle, a final extension step is performed at 72°C for 7 min. Then 5, ul of each PCR reaction is run on 2% agarose gel containing 5 Bg/ml ethidium bromide in Tris-acetate-EDTA buffer.

*RT-PCR for AFP* : Human AFP gene consists of 15 exons (Gibbs et al., Biochemistry, 26: 1332-1343). To distinguish truncated transcripts from functional complete AFP mRNA, two different portions of AFP cDNA sequence are selected as target molecules of RT-PCR. The primer combination of hAFP 1 and hAFP2 is used for the amplification of exon 1 containing the initiation MET to exon 3, whereas that of hAFP3 and hAFP4 amplify exon 12 to exon 14 containing the stop codon. The results of the PCR are shown in Figure 1. Both combinations of the primers result in strongly detected amplification bands in the RNA from Hep3B and HepG2 (lanes 1, 2, 4, and 5). By contrast, only the specific band of the C-terminal portion is detected by the primer set of hAFP3 and hAFP4 in the RNA from K562 (lanes 7 and 8). This result suggests that the erythroleukemia cell line, K562, expresses only a truncated form of AFP without the N-terminus. In support of this hypothesis, the PCR for the whole coding region of AFP using hAFP1 and hAFP4 primers is performed. As expected, the PCR of Hep3B and HepG2 cDNA shows the single remarkable band of 1. 8 Kb (lanes 3 and 6), whereas there is no band in K562 (lane 9). The controls are samples with no RNA and a sample derived from the mouse embryonic fibroblast cell line (STO). Neither shows any detectable band.

Next, a series of 5' primers from exon 2 to exon 6 are constructed to see the difference between authentic and variant form of hAFP mRNA. In Figure 1, the result shows that all the coding region except exon 1 is shared in the variant form of hAFP in K562 (lane 1, 3, 5, 7, 9, and 11).

*RT-PCR for albumin* : Human albumin gene consists of 15 exons also (Minghetti et al., J. Biol. Chem, 261: 6747-6757). As for AFP, the primer combination of hALB1 and hALB2 is used for the amplification of exon 1 containing the initiation MET to exon 4, whereas that of hALB3 and hALB4 amplify exon 12 to exon 14 containing the stop codon. The results of the PCR are shown in Figure 17. Both combinations of the primers result in strongly detected amplification bands in the RNA from Hep3B and HepG2 (lanes 1, 2, 4, and 5). By contrast, only the specific band of the C-terminal portion is detected by the primer set of hALB3 and hALB4 in the RNA from K562 (lanes 7 and 8). The PCR for the entire coding region of albumin using hALB1 and hALB4 primers show no band in K562 (lane 9). The controls are samples with no RNA and a sample derived from the mouse embryonic fibroblast cell line (STO). Neither show any detectable band.

### Suppliers for reagents include:

Sigma Chemical Company (St. Louis, Mo)
Gibco BRL Products (Gaithersburg, MD)
Worthington Biochemical Corporation (Frehold, New Jersey)
Dupont Pharmaceuticals (Wilmington, Delaware)
Falcon-a subsidiary of Becton Dickinson Labware (Franklin Lakes, New Jersey)

### Suppliers for tissues include:

Anatomical Gift Foundation (Atlanta, Georgia)
Advanced Biosciences Research, ABR (San Francisco, Cal)
Local transplant surgeons at UNC Hospital

### Example 2

### Processing of Human Livers

Fetal Livers : The fetal livers come from multiple clinics affiliated with Advanced Biosciences Research (ABR), all in California, or from the Anatomical Gift Foundation (AGF) with clinics in the South (i.e., Georgia, Virginia), Northeast (Pennsylvania) or Midwest (Kansas, Colorado). The fetuses are collected from clinics; the tissues dissected free from the fetuses and placed into RPMI 1640 (Gibco) supplemented with insulin (Sigma, 5, µg/ml), transferrin (Sigma, 5, µg/ml), selenium (10⁻⁹M, and 5% fetal bovine serum (Gibco). The samples are then put on ice and shipped by courier to our lab, a process that can take 10-16 hours. Thus, we receive the samples approximately 24 hours after surgery. The samples are assigned a number with the prefix REN, given in chronological order of being received (REN 1, 2, 3, etc), where REN is an abbreviation for Renaissance.

Adult Livers: The adult livers come from the Anatomical Gift Foundation or from local surgeons (UNC) and consist of rejected liver tissue, explants from transplant recipients, or livers donated for organ transplantation but then rejected for reasons other than pathogens. The patients providing explant tissue or rejected donor tissue are screened for an array of diseases and only those found safe by these tests are used for cell processing. After removal from the patients, the livers are put into University of Wisconsin solution (also called Viaspan) and shipped on ice to the lab. The time interval between organ removal from a brain-dead patient ("clamp time") and its arrival in the lab is extremely variable. The specimens arrive within less than 24 hours of "clamp time", the time at which the liver is removed from the donor.

Cadaveric Livers: Livers obtained postmortem within at least 30 hours of death are obtained through local organ procurement associations (*e.g*. Carolina Organ Procurement Association or COPA). The livers are processed as for the adult livers.

The list of elements checked for investigator's safety is : HIV I and II, HTLV I and II, hepatitis B and C; tuberculosis. The list for clinical usage is : HIV I and II, HTLV I and II; hepatitis A, B, C, and G; EBV, CMV; tuberculosis, syphilis and mycoplasma.

Fetal and adult livers are processed using a combination of enzymatic digestion and mechanical dissociation, fetal livers are prepared primarily by mechanical dissociation, whereas the adult livers are dissociated primarily by enzymatic digestion. A description of each is given below. Both fetal and adult livers are digested for varying lengths of time in an enzyme buffer that serves to dissolve the extracellular matrices that bind the cells together in a tissue. The collagenase enzyme mix used for isolation of liver cells is a high purity "Liberase" enzyme preparation manufactured by Boehringer-Mannheim, consisting of a mixture of purified collagenase and elastase. This enzyme mix can be used at much lower concentrations and with fewer deleterious "side effects."

**Enzyme solution** : collagenase solution--60-70 mg/100 mls of buffer (Sigma's type IV collagenase, catalog #C5138 or Worthington's type B, catalog #LS005273; both being bacterial preparations enriched in collagenase but with many enzymatic impurities) or Liberase-- (purified collagenase/elastase preparation by Boehringer-Mannheim, catalog 1814184) prepared in P2 buffer (see below) and used at 0. 23 mgs/ml

**Cell Wash Solution** : RPMI 1640 (Gibco) supplemented with insulin (5, ug/ml), transferrin (5 ug/ml), free fatty acid mixture (see below) bound 1 : 1 molar ratio to purified bovine or human serum albumin.

**Free Fatty Acid Mixture**: Immature cell populations, and damaged older liver cells, require lipids to maintain and to synthesize their membranes. Although fully mature hepatocytes can synthesize their membranes from a single fatty acid source (linoleic acid) younger parenchymal cells cannot and thus require a mixture of many different fatty acids to handle their lipid requirements. We provide a complex mixture that is then bound in a 1: 1 molar ratio with a highly purified albumin. A detailed description of the method for preparation of that fatty acid preparation is given below :

The stock solutions are prepared as follows, for a combined total of 100 mM free fatty acids :

| | | | |
|---|---|---|---|
| Palmitic | 31.0 mM | Oleic | 13.4 mM |
| Palmitoleic | 2.8 mM | Linoleic | 35.6 mM |
| Stearic | 11.6 mM | Linolenic | 5.6 mM |

To obtain a final concentration of 7. 6, µM/L, add 76, µl per liter. [REF : Chessebauf and Padieu, In vitro 20 (10) : 780: 1984. According to the above reference a mixture of free fatty acids is used at a final concentration of 7.6, µeq/L (=7. 6, µM) in cell culture media.]

### Preparation of the Individual Fatty Acid Components:

Each individual component is dissolved in 100% EtOH as follows :
Palmitic 1 M stock, soluble in hot EtOH
Palmitoleic 1 M stock, readily soluble in EtOH
Stearic 151 mM stock, soluble in heated EtOH at 1 g/21ml
Oleic 1M stock, readily soluble in EtOH
Linoleic 1 M stock, readily soluble in EtOH
Linolenic 1 M stock, readily soluble in EtOH

These individual stocks are then mixed to obtain the 100mM FFA mixture. Aliquots of the individual FFAs and the FFA mix were made with bubbling nitrogen through to reduce oxidation and increase stability. Stocks are frozen at-20 °C.

**PI Perfusion buffer**--calcium and magnesium free perfusion buffer (pH 7. 2) with final concentrations as specified for each of the following components : 118 mM NaC 1, 4. 7 mM KCl, 1. 2 mM KP0⁴, pH 7. 4, 2. 5 mM NaHC03, 0. 5 mM EDTA, 5. 5 mM glucose, 0. 5% bovine or human serum albumin (BSA), Ascorbic acid (50, µg/ml), insulin (4, µg/ml), dexamethasone (µM).

### P2 Perfusion buffer--Dulbecco's modified Eagle's medium or RPMI 1640 supplemented with 0.5% BSA, ascorbic acid (50 µg/ml), insulin (4, µg/ml) and dexamethasone (1 µM).

**DMEM**--Dulbecco's Modified Eagle's medium (Gibco) with glucose, sodium pyruvate and L-glutamine and further supplemented with 5% fetal bovine serum, insulin (4, µg/ml) and dexamethasone (1µM).

**Chee's medium** supplemented with ITS^{+™} culture supplement (5 mls/500 mls) and dexamethasone (0. 1, µM)

**Percoll** (Pharmacia, catalog # 17089102) is diluted 9:1 with 10X Dulbecco's phosphate buffered saline.

### Example 3

### Fetal Liver Tissue Studies

The fetal livers arrive in the transport buffer (described above) and on ice. They are rinsed with a"cell washing buffer"consisting of RPMI 1640 (Gibco) supplemented with insulin (Sigma; 5 µg/ml), transferrin (Sigma; 5, µg/ml selenium (Johnson Matthey's mass spec trace elements; 10^{-9M}), and a free fatty acid mixture bound to bovine serum albumin in a 1:1 molar ratio. The fetal livers are then put into a collagenase buffer for 15-20 minutes and then gently pressed through a "collector" (Sigma) with an 800 mesh grid to yield small aggregates of cells; the "cell wash buffer" is used to facilitate the dissociation process. The aggregates of cells are then fully dissociated by pressing them through a 70 Micron filter (Falcon cell strainer, 70 µm nylon, catalog #2350) using the "cell wash buffer" to facilitate the process. The cells that pass through the 70 micron filter are kept separate from those that do not. Both samples are cryopreserved and checked for percentage viability using the Trypan blue dye exclusion assay.

### Example 4

### Adult Liver Tissue Studies

The livers are catheterized by the portal vein, vena cava, or by both, perfused with buffers to eliminate blood; and then perfused with buffers containing collagenases/proteases to enzymatically dissociate the cells. After the digestion, taking usually 15-30 minutes depending on the size of the liver, the tissue is pressed through cheesecloth or a nylon filter or raked with a comb to mechanically complete the cell dissociation process. The dissociated cells are rinsed with a buffer containing serum to inactivate the collagenase and other enzymes used in the perfusion process.

The perfusion buffers, PI and P2, are placed in a water bath at 37°C. The perfusion is carried out in a Miller type perfusion box, which is maintained at 37°C throughout the perfusion. The buffers are oxygenated during the perfusion. All tubing in the box is rinsed with 70% ethanol, followed by distilled water and then with PI to ensure that the air has been removed from the system. The liver is cannulated using a Teflon cannula from a 16-gauge needle attached to 60 ml syringe to flush ice-cold PI buffer through the liver using various blood vessels available on the cut surface of the liver for large pieces of liver (100-300 gms). For the rare cases when an entire liver lobe becomes available, the remnants of the vena cava can be cannulated. The various blood vessels in chunks of liver are tested to learn which will offer optimal perfusion of the tissue. This procedure also removes any excess blood from the liver. The chosen blood vessel is cannulated and sealed into place using medical grade adhesive (medical grade "superglue"). All other large vessels and surface openings are sealed using the medical grade adhesive, and, if required, using Q-tips with the adhesive to help seal the openings. Once the adhesive has dried, the liver specimen is placed on a nylon mesh within an appropriate size glass bowl. The PI buffer is added to the bowl, and the liver submerged in the buffer. The bowl containing the liver is placed inside the perfusion box, and the outlet tubing of the cannula is attached. The PI buffer is recirculated for 15 minutes starting at a low speed of about 24 mls/min and then slowly increased to between 58 mls/min and 90 mls/minute to optimize a flow rate with an acceptable back pressure. One must check that there are no excessive leaks of the perfusate from the liver. After 15 minutes, the PI buffer is removed from the bowl and replaced with the P2 buffer containing the collagenase. The P2 buffer is recirculated until the liver is sufficiently digested (evaluated by color- conversion of liver from dark reddish brown to pale brown and by acquisition of mushy texture to liver). The P2 buffer is recirculated for no longer than 20-25 minutes. Once the perfusion has ended, the P2 buffer is drained from the bowl and the liver transferred in the bowl to a biological hood.

The cell culture medium (DMEM) is added to the bowl, and the cannula and the adhesive is removed along with any undigested regions of the liver. The capsule of the liver (Glisson's capsule) is broken using tissue forceps and scissors. This allows the release of the digested tissue into the medium leaving behind the connective tissue and any undigested material. The digested material is put into the DMEM and then filtered through a series of different size filters. The filters are placed inside a large funnel to aid the filtration. The digested material is filtered first with a single layer of cheesecloth, followed by a 400, µm nylon filter, and finally through a 70 µm Teflon filter. The filtrate is divided equally into centrifuge tubes and centrifuged at 70 g for 4 minutes.

After centrifugation, prior to the addition of Percoll, the supernatant is referred to as the Fraction 1 (F1). To the pellet of cells, DMEM and isotonic Percoll are added to give a final ratio of 3:1 respectively. For example, a small pellet of packed cells of 5 ml volume would be suspended in 30 mls of DMEM and 10 mls of isotonic Percoll. The sample is centrifuged at 100 g for 5 minutes. The supernatant is obtained : the top layer is referred to as Fraction 2 (F2). The middle layer of the Percoll is referred to as Fraction 3 (F3). The pellet of cells that remains is Fraction 4 (F.). The cells of the different fractions are suspended and assessed for viability using the Trypan blue dye exclusion assay. The viabilities of these different fractions are presented in Table 3, along with their viabilities after cryopreservation.

Cells that remained bound to the vascular or biliary tree of the liver tissue following liver perfusion were retained. These cells are found in the original suspension of cells obtained after enzymatic perfusion, and are typically left on the top of the sieves (*e.g*. cheesecloth) after passing through the cells in suspension. These remnants of the vascular and biliary tree are processed again with enzymes and the resulting cells pooled together with the other cells.

Percoll fractionation is used routinely by most investigators to eliminate what they assume to be debris and dead cells; only the final pellet is preserved. The novel variation to the perfusion routine, as disclosed herein, is that the pellet was discarded and cells with a lowest buoyant density (i.e., cells collecting at the top of the gradient) are being retained and used for further studies. These cells are younger parenchymal cells and have a much greater ease of freezing (see section on cryopreservation).

### Example 5

**Cryopreservation Experiments.** The livers used for cryopreservation methodologies have derived from donors as young as fetal livers (gestational ages 12 weeks to 25 weeks) and as old as 77 years of age.

### "Novel Cryopreservative buffer"

• Viaspan (Dupont Catalog # 1000-46-06) supplemented with 2% human serum (Gibco) or fetal bovine serum (Biowhittaker),
• 10% cryopreservative [dimethylsulfoxide (Sigma catalog #D5879 or D8779) used exclusively for mature parenchymal cells or dimethylsulfoxide or glycerol (Sigma catalog # G6279) used for progenitors].
• The buffer is further supplemented with antibiotics (penicillin at 200 U/ml; streptomycin at 100, µg/ml),
• The buffer is further supplemented with hormones and growth factors: insulin (5, µg/ml), transferrin (5, µg/ml), epidermal growth factor (50, µg/ml), FGF (10 ng/ml), IGF II (10 ng/ml),
• The buffer is further supplemented with lipids : free fatty acids (7. 6, UM/1) bound to bovine serum albumin (BSA) or human serum albumin (HSA) and high density lipoprotein (10, µg/ml)
• The buffer is further supplemented with trace elements (selenium (10-9M), copper (10⁻⁷), zinc (5 X 10⁻¹¹ M)) and an antioxidant, (*e.g*. a porphorin that is a superoxide dismutase mimetic, used at 10, µg/ml; ascorbic acid, used at about 0.1 mg/ml; or any antioxidant known in the art).

The variation in the composition, as disclosed herein, is to combine the key nutrients, lipids, hormones and growth factors that were identified as part of serum-free hormonally defined media tailored for liver cells. The novel buffer results in viabilities of the liver cells for the F4 fractions that are as low as 50% (from very poor samples) to as high as 80% (for good samples). The viabilities of the FI-F3 fractions are consistently above 80%, a fact that we suspect is because these fractions have younger cells with ploidy states and metabolic activity more conducive to synthesis of extracellular matrix components and/or other cellular factors needed for viability and growth; thus, they are likely to be easier to freeze. The use of a superoxide dismutase mimetic in the buffer increased the viability of the cells by 5-10%.

### An alternative to the above is to :

• use a modified buffer in which the Viaspan is eliminated and the basal medium (such as RPMI 1640) is supplemented with insulin (5 ug/ml), transferrin (5 µg/ml), free fatty acids (7. 6, µM/1) bound to BSA, high density lipoprotein (10, µg/ml), trace elements (selenium (10⁻⁹M), copper (10⁻⁷M), zinc (5 X 10¹¹ M)), and an antioxidant
• coat the cells with a form of extracellular matrix such as type IV collagen mixed with laminin, or type I or type III collagen mixed with fibronectin.

Fetal liver cells, processed as described above, are suspended in the cryopreservation buffer (described above), aliquoted into 3 ml cryovials at 5-10 X 10⁶ cells/ml and maintained under that condition for 1-2 hours. The cells are then frozen to liquid nitrogen temperatures of -100°C to -180°C, preferably -160°C using a computerized control rate freezer (Forma Cryomed) and then stored in a large vapor phase, liquid nitrogen (-160°C) storage tank. Cells survive the process well and no significant loss of viability occurs over storage periods ranging from 50-270 days (see Fig 3).

The fractions of adult liver cells (F1-F4) were found to contain distinct cell populations : F1 contains debris, red blood cells, hepatic stellate cells, and small hepatic cells (<10, µ) that are probable progenitor cell populations (of either hepatic or hemopoietic lineages); the F2 fraction, the top of the Percoll solution, contains larger hepatic cells (10-15, µ) that are diploid, small parenchymal cells; the F3 fraction at the bottom of the Percoll contains yet larger parenchymal cells (15-25, µ) consisting of a mixture of diploid and tetraploid cells; and the F4 fraction (the one used by all other investigators) consisting of the largest of the parenchymal cells (25-50 µ) and that are entirely polyploid (tetraploid and octaploid). In general, the parenchymal cells in the F1-F3 fraction have a viability after freezing of 85-95%; the parenchymal cells in the F4 fraction have a 50-80% viability after freezing (depending upon the conditions of the liver upon arrival). The identified variables influencing viability of the parenchymal cells in the F4 fraction are: 1) age of the donor (the older the age of the donor, the worse the prognosis for the cells); 2) the time between" clamp time" and delivery to the lab (the shorter the better); 3) health status of the liver tissue prior to removal (*i*.*e*., severe ischemic condition confers a bad prognosis). These factors are interactive such that rapid delivery of tissue from an elderly donor may be more attractive than tissue from a young patient that has spent too long in transit.

**Table 5. The average viabilities and attachment efficiencies of fetal and adult livers with cryopreservation and the % of hepatic progenitors (AFP+ cells) in the cell suspension.**

| Cell Population | Cryopreservative | Viability after processing | Viability after thawing | Ave. Cell Size (in um) | Growth in culture | %AFP+ cells |
|---|---|---|---|---|---|---|
| Fetal livers | Glycerol | 76% | 77% (i.e. 100% of recovery | 7-15 | good | 6-7% |
| Adult Liver, F1 | Glycerol/DMSO | 80% | 82-85% | >12 | good | 0.5-1% |
| Adult Liver, F2 | Glycerol/DMSO | 85% | 84% | 12-15 | good | 2% |
| Adult Liver F3 | DMSO | 85% | 85% | 15-25 | good | 0.2% |
| Adult Liver, F4 | DMSO | 50-75% | 565 | 25-50 | poor | 0.01% |

The extreme range of viabilities of the F4 fractions both after processing and after freezing are due to the varying lengths of time between "clamp time" and receiving the samples in the lab and also to the varying conditions of the liver (fibrotic, ischemic, etc.). In general, the F4 fraction is the most sensitive to the vagaries of treatment of the livers and the general health of the tissue. Remarkably, the F2 and F3 fractions were routinely viable and readily cryopreserved even when obtained from poor liver specimens. The F 1 fractions were more variable, containing a large amount of debris, fat droplets as well as numerous small cells that included both small parenchymal cells (assumed to include hepatic progenitors) and various hemopoietic subpopulations (i.e., erythrocytes).

**Table 6. Cryopreservation : Fetal Liver**

| **● >200 processed** | | **● Yield** | |
|---|---|---|---|
| **● Tissue received** | | | **◆~ 10⁸ cells per gram** |
| | **(by donor age)** | | **processed tissue** |
| | **◆ 12 wks: ~ 1 ml packet** | **● Viability** | |
| | **Cells** | | **◆ Processing: 75-85%** |
| | **◆ 16 woks: ~ 15-20 mls** | | **◆ Thawing: >95%** |
| | **packed cells=0.5-1 gm** | | **◆ Sorting: >90-%** |
| | **tissue** | | **◆ In culture: >90%** |
| | **◆ 24 wks: ~ 4-5 gms** | | |

**Table 7. Cryopreservation: Adult Liver**

| ● **>80 processed** | | **● Viability (processing)** | |
|---|---|---|---|
| ● **Received 100-200** | | | **◆ F1:>75%** |
| | **• Grams per liver** | | **◆ F2: >90% (12-15 µ)** |
| | **(of 2.5-3 kg/liver)** | | **◆ F3: >90% (15-25 µ)** |
| | **• Yield:** | | **◆ F4: 75-80% (25-50 µ)** |
| | **10⁷⁻10⁸ cells per gram** | **● Viability (freezing)** | |
| | | | **◆ F1-F3: .80%** |
| | | | **→ good attachment** |
| | | | **◆F4: 56%** |
| | | | **→ fair attachment** |

### Example 6

### Flow Cytometry

The cells are passed in single file through a flow cell where they are exposed to laser light. The approximate volume of each cell is determined by "forward scatter", or the amount of light that is refracted as the beam is intersected. Scattered light, "side scatter" from internal cellular structures such as the nucleus, endoplasmic reticulum Golgi bodies, vesicles, etc., are used to determine the amount of internal complexity (i.e. an active cell and a more mature cells win contain more internal components than a quiescent one or a younger one). More selective information on cell characteristics is obtained by binding highly specific, characteristic antigens to protein complexes on the cell surface. These antibodies can be covalently bonded to fluorescent molecules such as Fluorescein Isothiocyanate (FITC), Phycoerythrin (PE), and tandem conjugates of PE and Cytochrome which are excited by the laser beams, generating emitted light at specific wavelengths for each fluorophore. By selecting a panel of distinctive chromophores conjugated to specific antibodies cell populations of interest are selected.

Cells are analyzed based on their parameters input. A variety of collection devices are used to collect the desired cells, including Eppendorf and conical tubes, and any size multi- well plate at the speed of up to 40, 000 events per second or higher.

### Antibodies and reagents used in staining procedures

| | | |
|---|---|---|
| Antibody | Supplier, | Cat #; Lot # |
| Goat anti-human AFP | Chemicon, | AB635, C4P168 |
| Monoclonal mouse X human Thy | Chemicon, | MAB1294,293CCD |
| Monoclonal mouse antihuman | | |
| AFP-PE conjugate | Chromaprobe, | P41020, A45P7 |
| Biotinylated Rabbit anti-Goat | Vector Laboratories, | BA-5000, J0313 |
| Biotinylated Rabbit anti-Goat, Jackson Immunochemicals 200-152-096, 25985 | | |
| Streptavidin/AMCA conjugate, Jackson Immunochemicals, 016-150-084, 40001 | | |
| Donkey anti-sheepAMCA conjugate, Jackson Immunochemicals, 713-156-4732202 | | |
| Donkey anti-Goat CY5 conjugate, Jackson Immunochemicals, 705-156-147, 38756 | | |
| Goat IgG, Jackson Immunochemicals, 005-000-002, 38837 | | |
| Sheep IgG Jackson Immunochemicals, 013-000-002, 39945 | | |
| Sheep anti-human Albumin, Serotec, ABP102, 210498 | | |

**Mouse monoclonal anti-human:**

| | | |
|---|---|---|
| CD14/Tri Color conjugate | Pharmingen | |
| ICAM | Pharmingen | |
| CD34/FITC conjugate | Pharmingen | 34374X |
| CD38/PE conjugate | Pharmingen | 31015X |
| CD38/FITC conjugate | Pharmingen 31014X | |
| Glycophorin A PE conjugate | Pharmingen | 32591A |
| CD 45/PE conjugate | Pharmingen | 31255X |
| CD 45/FITC conjugate | Pharmingen | 31254X |
| Isotype controls IgGIPE | Pharmingen | 33815X |
| IgG2 FITC | Pharmingen | 33814X |
| c_ Kit PE conjugate | Caltag | MHCK04 |
| Rabbit X Human AFP-FITC conjugate Accurate | YNRH AFPF not listed | |
| Goat anti-Human AFP unconjugated | AXL625 | 061 |
| 7 Amino Actinomycin D | Mol Probes A-1310, 4981-1 | |
| (7AAD) | | |

### Principal solutions used in cell preparations for flow cytometry :

BSA : bovine serum albumin (Pentex V)
PBS = phosphate buffered saline ;
FBS = fetal bovine serum ;
AFP = alpha-fetoprotein

### Dulbecco's Modified Eagles Medium with Hormones : HC DMEM

500 mL DMEM, high glucose without phenol red
25 mL fetal bovine serum (FBS)
20 mL 5mM EGTA
Insulin (5 µg/ml), transferrin (5, µg/ml)
Trace elements [selenium (10⁻⁹M), copper (10⁻⁷M), zinc (5 X 10⁻¹¹ M)]
Antibiotics (Penicillin-100, µg/ml, streptomycin-100, µg/ml)
500 mg bovine serum albumin (BSA) 30 mg DNase
38, µL free fatty acid mixture bound to BSA.
Sterile filtered through a Nalgene filtration unit with 0.2, µm pores

### Hanks Buffered Saline Solution-modified version : HBSS-mod

50 Ml 10X HBSS
10 mL 1MHepes
Penicillin-100 µg/ml/µg/ml/Streptomycin-100 µg/ml
500mg BSA
30 mg DNase
Make up to 400 mL
pH to 7.3
Top up to 500 mL
Sterile Filter at 0.2 µm

### Blocking buffer for immunochemistry

100mlsofHBSS_mod
2. 2 mL 45% teleostean fish gel and
0. 8g BSA
0. 5mL 1% saponin in HBSS

### Mounting medium for Immunofluorescent microscopy

0.5 mL 2X PBS
0. 25g n-propyl gallate
5. 7g glycerol

### Example 7

### Procedures for preparation of frozen liver tissue for flow cytometry

Thaw frozen liver tissue rapidly at 37°C. Each cryovial of liver (each containing about 3 mL of buffer containing 5-10 X 10⁶ cells/mL) is brought up to 10 mL at a rate of 1 mL per min. on ice with HC-DMEM. The sample is then centrifuged at 1200 RPM for 5 min at 4°C. The supernatant is discarded, and the pellet of cells resuspended in 5 mL of HC-DMEM. The washing of the cells is repeated until the supernatant becomes clear. Then the cells are counted and the viabilities assessed with a hemocytometer using the trypan blue dye exclusion assay. The cells are split into fractions according to the experimental protocol. Standard tubes are prepared for control data containing between 1 and 2 X 106 cells, usually achieved by taking 200, uL for each from a cell suspension of 5-10 X 106/mL. The following standard tubes are needed:
1) OCS. Original cell suspension which consists of unstained control cells.
2) FITC alone for compensation adjustments. Add 5, µL of FITC-labeled anti glycophorin A to 200, µL of cell suspension. Alternative is a cocktail of FITC-labeled CD34, CD38 and CD45, 7 µL of each into 200 µL of cells.
3) PE alone for compensation adjustments. Use a Glycophorin-PE (2 µL to 1 mL HC_DMEM and add 30, µL of this to 200, µL of cells).
4) 7AAD alone for compensation. A good signal is generated by fixing 200, µL of cell suspension with 2% paraformaldehyde and then adding 5, µL of 100 µM 7AAD and 5 µL of detergent (1 % saponin) to a 1 mL suspension of these cells in HBSS-mod. The permeabilized cells stain intensely with 7AAD.
5) Cy5 alone for compensation 200 µL of fixed cells (2% paraformaldehyde) are incubated for 40 min in 2% goat serum to label the cell surfaces with sheep IgG. The cells are then incubated with Cy5 conjugated donkey anti-goat IgG (1 : 800) for 40 min.
6) AMCA alone for compensation. As with 7AAD, an artificially intense signal is generated for compensation adjustments. 200 µL of fixed cells (2% paraformaldehyde) are incubated for 40 min in 2% sheep serum to label the cell surfaces with sheep IgG. The cells are then incubated with AMCA conjugated donkey anti-sheep IgG (1 : 800) for 90 min.
7) AMCA/Cy5 controls. Incubate fixed (2% paraformaldehyde) and permeabilized (0. 05% saponin) cells with AMCA-conjugated donkey anti sheep IgG and Cy5- conjugated donkey anti goat IgG for 90 min.
8) Monoclonal Isotype controls. Incubate cells with a mouse IgGI PE conjugate and a mouse IgG2 FITC conjugate. Concentrations should match those used to label analytical and sort tubes.
9) Intracellular Isotype Controls. Incubate fixed (2% paraformaldehyde) and permeabilized (0. 05% saponin) cells with non-immune sheep IgG and goat IgG for 90 min as controls for antibodies used for identification of albumin and alpha-fetoprotein. Continue with incubation with Cy5-conjugated donkey anti-goat IgG and AMCA-conjugated donkey anti sheep IgG for 90 min.

Sort tubes are prepared for the acquisition of selected cell populations expressing particular combinations of CD markers. Normally these tubes contain 50-70 X 106 cells. Cells are resuspended in 1 mL of staining buffer comprised of HC_DMEM + 1% BSA + 500 pM 7AAD (5 uL of 100 mM stock). Between 15 and 25, LL each of CD 34 FITC, CD38 PE, or CD 45 PE are added to the staining buffer according to cell numbers (normally 3, µL of Pharmingen antibody per 10 X 10⁶ cells). Antibody to c-Kit is added at a 1 : 60 dilution, glycophorin A is used at a 1 : 500 dilution. Stain for 40 min on ice in the dark. After staining wash cells twice with HBSS-mod and fix with 2% paraformaldehyde in PBS for 30 min on ice.

### Example 8

### Intracellular Staining For Cell sorting

For intracellular staining of cells for analysis of alpha-fetoprotein (AFP) by flow cytometry the cell suspension is permeabilized with a mixture of saponin (Sigma S4521) 0. 05% in HBSS_mod for 10 min on ice. Cells are then blocked in a mixture of HBSS_mod containing 1% teleostean fish gel and 0. 8% BS and 0. 005% saponin for 20 min, followed by incubation with goat anti-human AFP and sheep anti human albumin (both 1 : 800 in blocking buffer) for 90 min at room temperature in the dark. Cells are washed twice with HBSS mod containing 0. 01% saponin followed by incubation with Cy5-conjugated donkey anti-goat IgG and AMCA-conjugated donkey anti sheep IgG for 90 min.

Alternatively, following the primary antibody, cells are incubated with biotinylated rabbit anti goat IgG (1 : 500 in blocking buffer containing 2% human serum and 0. 01% saponin for 90 min at room temp in dark). This is followed by 2 washes with HBSS_mod containing 0.01% saponin and then incubation with 9 ug/mL streptavidin/Cy5 conjugate in 0. 01% saponin/HBSS_mod for 90 minutes at room temperature in dark. Finally, cells are washed 2 times with HBSS_mod and resuspended in HBSS_mod, filtered though a 50, um sieve to remove clumps of cells for analysis and sorting on the flow cytometer.

If selection of hepatic progenitors is intended, the immunoselection includes removing cells that are polyploid and/or express markers associated with mature hemopoietic cells from the liver such as glycophorin A on red blood cells. Additionally cells exhibiting CD45, which is expressed on all mature hemopoietic cells are all removed.

### Example 9

### Immunohistochemical Staining of Sorted Cell Populations.

Cells are stained for alpha-fetoprotein after analysis and sorting by the flow cytometer. The sorted cell fractions are collected in 0. 3% HBSS-mod containing 1% BSA. Upon return to the laboratory the volume of collected samples is adjusted to provide 0.5 X10⁶ cells/mL and 200 uL aliquots are spun onto microscope slides with a Shandon Cytospin apparatus. The cytospun slide preparations are air dried and stored for later staining for alpha-fetoprotein and/or albumin. The attached cell "disk" of the microscope slide are ringed with a rubber dam to produce a "well" for application of immunohistochemical reagents. Slides are soaked in tris buffer ("low salt" 10 mM tris with 0.9% NaCl at pH 7. 4) containing 0 3% Triton X for 10 min, followed by 10 min in low salt Tris alone.

Cells are then blocked in 10% rabbit serum contained in a teleostean gel blocking solution described above for 90 min at room temperature. After two washes in low salt Tris, cells are incubated overnight at 4 °C with goat anti-human AFP antibody diluted to 1:100 in blocking buffer containing 2% rabbit serum. Two washes in Tris buffer are then followed by a 90 min incubation with biotinylated rabbit anti goat IgG (1 : 200) in blocking buffer at room temp. Final incubation with streptavidin/AMCA complex (9, µg/mL in low salt Tris buffer) is used to locate AFP-like immunoreactivity through binding of the AMCA fluorochrome with the biotinylated rabbit antibody. Following 2 washes with Tris buffer the cell preparations are allowed to come close to dryness before coverslipping under an antifade mounting medium (0.25g n-propyl gallate in 5.7g glycerol with 1 mL PBS). When appropriate, cells are double-stained for albumin by including a Texas red conjugated rabbit anti human antibody against albumin with the primary anti-fetoprotein antibody.

Control slides are prepared by omission of the primary or the secondary antibody to demonstrate no AMCA labeling of cells in the absence of either the anti alpha protein antibody or the biotinylated secondary antibody. Slides are inspected with epifluorescence microscopy using UV excitation of the AMCA dye which emits light in the blue (450 nm) region.

### Example 10

### Cell and/or gene therapy.

Since human urokinase plasminogen activator (uPA) can activate plasminogen across species a recombinant adenoviral vector that expresses human urokinase from the RSV-LTR promoter, Ad-RSV-uPA is constructed with the aim to induce liver regeneration. For construction and production of the recombinant adenoviral vectors, the cDNA for human uPA is prepared as follows. The 1. 326 kb Hindlil/Asp718 uPA fragment that contains the protein coding sequence is insetted into the Hindill/Asp718 sites of pXCJL. 1 under the transcriptional control of the Rous Sarcoma Virus LTR (RSV) promoter, and upstream of the bovine growth hormone polyadenylation signal. The virus is prepared after co-transfection with pJMI7 and the vector designated Ad-RSV-uPA. The screening for Ad-RSV-uPA is carried out by amplification of individual plaques in 293 cells. Three days after infection the supernatant is tested for immunological reactive uPA by ELISA and fibrinolytic activity by fibrin plaque assay demonstrating the catalytic activity of uPA produced upon Ad-RSVuPA infection. The purified virus is stored in aliquots at-80 °C and freshly diluted with HGDMEM media prior to injection. The viral titers are determined by OD measurements and standard plaque assay. The construction of the vectors is essentially carried out as described in the U. S. Pat. No. 5, 980, 886. The viruses are titered on 208F cells.

C57BL/6 female mice aged 5 to 6 weeks (Jackson Laboratories, Bar Harbor, ME) are housed in a specific pathogen free environment. Ischemic liver samples at various time periods are obtained from euthanased mice and liver progenitors are isolated as disclosed supra. For portal vein cannulation, recipient mice are anesthetized by an intraperitoneal administration of 0. 5 ml of 20 mg/ml 2, 2, 2-Tribromoethanol. A midline abdominal incision is made and the skin is separated from the peritoneum to create a subcutaneous pocket. The peritoneum is opened and the portal vein is exposed. A silicone tube (0. 02" I. D., 0. 037" O. D., S/P Medical Grade, Baxter, 111.) is inserted in the portal vein and perfused with heparinized saline. Thereafter the cannula is tunneled through the peritoneum and secured with a 4. 0 silk suture. The 3cm long cannula is tied off at the distal end and placed subcutaneously in the previously created pocket. The mice are given the virus-infected progenitor cells no earlier than 24 hrs later. In some mice the portal vein cannulation is performed together with a 2/3 hepatectomy. The partial hepatectomy is then carried out. To perfuse the portal vein, mice are anesthetized, the skin is opened at the proximal site of the already existing abdominal incision. The cannula is exposed and connected to a syringe pump. For virus infusion, the preps of adenovirus in DMEM are injected over 5 to 10 min into the portal vein through the cannula.

All biochemical and histological analysis are performed after injection of adenovirus-infected hepatic progenitors into the portal vein through the cannula. The ELISA assay for uPA is based on two different monoclonal antibodies directed against the catalytic and receptor-binding domain of uPA. One of the monoclonal antibodies is labelled with peroxidase. Serum total protein and albumin are analyzed by routine automated methods in the clinical pathology laboratories. Infusion of adenovirus into the portal vein of C57BL/6 mice is known to result in transduction of 1 00% of hepatocytes with more than 1 copy of adenoviral DNA per cell. The same dose of Ad-RSV-uPA results in 90% mortality that at least in part was related to hemorrhage. When lower dose of Ad-RSV-uPA is used, the mortality rate is less than 5% and this dose is selected for the liver regeneration experiments. The infusion of Ad-RSV-uPA results in transient elevations of serum urokinase reaching a peak value of about 350 ng/mi (70 to 100 times greater than endogenous levels) four days later before failing to background concentrations by day 12. The rise in uPA is also associated with an increase in the serum SGPT concentrations. At varying times after adenovirus infusion, animals are infused with 3H-thymidine, and the amount of radioactivity incorporated into liver DNA is determined as a means to quantitate cell proliferation. The animals treated with Ad-RSV-uPA had an increased period of thymidine uptake that began on day 3 and persisted for 8 days. Thus, the period of hepatic 3H-thymidine uptake with Ad-RSV-uPA/oval cells treatment is much greater than that obtained with partial hepatectomy. The recipients of the negative control adenovirus show peak of hepatic 3H-thymidine uptake on day 4 that returned to baseline levels 24 h later and a minimal rise in 3H-thymidine uptake on day 11. In summary, the hepatic damage as measured by SGPT levels and high rates of 3H-thymidine uptake is attributed to intrahepatic urokinase production indicating that significant liver biosynthetic regeneration occurs. Hepatic progenitor cells infused without uPA are better than adenovirus without uPA insert.

Microscopic histological findings from animals treated with recombinant adenovirus/progenitors derived from non-heart beating cadaver donors indicate that by day 3 treated mice had a moderate inflammatory infiltrate that contained macrophages and neutrophils. Degenerative changes in hepatocytes included vacuolization, pyknotic and few mitotic nuclei. Eight to 10 days after Ad-RSV-uPA/oval cell administration there is evidence of hepatic recovery including the presence of multifocal regeneration, heterogenous size of nuclei, and a much decreased inflammatory reaction with few degenerating hepatocytes. By three to four weeks, the infiltrate resolved and the liver appears normal.

In total, these studies demonstrate that urokinase expression in combination with hepatic progenitors induced significant liver parenchymal cell regeneration.

### Example 11

### Debulking by Percoll Centrifugation

This example provides methods for enrichment of liver progenitors, in including liver stem cells, uncommitted progenitors, and committed progenitors. Variations of these techniques are known to those skilled in the art and are equally suitable as long as they are agreeable with the goal of debulking liver cell suspensions to provide an enriched population of progenitors.

A substantially single cell suspension of liver cells in culture medium, *e.g.* the basal medium of Eagle (BME), is applied to the top of a layer of 15% Percoll prepared in BME. Using a Sorvall RT7 centrifuge and a 14 cm rotor, or other equivalent rotor centrifuge combination, the gradients are centrifuged at 600 to 1200 rpm, preferably 750 to 1000 rpm for 10 min. The supernatant is collected and centrifuged again, but at 1200 to 2000 rpm, preferably about 1500 rpm. The supernatant fraction is enriched in progenitors and the pellet (F3 fraction) contains cells capable of at least one cell cycle. The supernatant cells are collected separately and centrifuged again, at 2000 to 3000 rpm, preferably about 2500 rpm. In this latter centrifugation, progenitor cells frequently sediment into the upper regions of the Percoll, leaving cell debris at the upper levels, and the pellet has cells capable of several cycles of mitosis. The Percoll fraction is suitable for immediate use, cryopreservation, establishment in culture, or further enrichment. Further enrichment can be accomplished by panning, affinity selection, FACS sorting or any of the techniques known in the art and described above. Negative selection is accomplished by removal of cells expressing markers for CD45, glycophorin A . Positive selection is accomplished by selection of cells expressing CD14, CD34, CD 38,

### Example 12

### Preparation of Progenitor Cells by Elutriation

This example provides steps for an isolation of committed and uncommitted liver progenitor cells. While various techniques are known in the art, one of the preferred embodiments is disclosed in detail with understanding that other preparation techniques are equally suitable as long as they are agreeable with desired goals. For examples of preferred, nonlimiting techniques see for example U. S. Pat. Nos. 5,807,686, 5,916, 743,5,672, 346, 5,681, 559, 5,665,557, 5,672,346, and 5,663,051.

Pluripotent or committed hepatic, small liver cells can be preliminary isolated using either Percoll, or other suitable density gradients such as Histopaque, and after centrifugation, washed twice with media and resuspended in 10 ml of elutriation media. For counterflow elutriation, the washed small mononuclear cells are injected via a sampling site coupler into the inlet stream of a Beckman J6M/E centrifuge equipped with a JE-5 rotor and standard chamber. However, any of a number of commercial continuous flow centrifuges and elutriators that preferably employ disposable plastic insets including chamber means for facilitating density based separation can be used, such as the "Fenwal Models CS 3000" and "Autopheresis C" sold by Baxter International Inc, of Deerfield, IL; or Spectra Apherisis v 7/6, sold by Cobe manufacturing of Lakewood, CO. The choice of instruments is up to one skilled in the art. A peristaltic pump (Cole Palmer Instruments, Chicago, IL) provides continuous flow of elutriation medium, which is 0.9% normal saline solution with 100 mg/dl D-glucose, 0.3 Mm disodium ethylenediaminetetraacetic acid (EDTA) and 50 mg/dl bovine serum albumin with pH adjusted to 7.2. The medium is sterilized prior to use. Cells are delivered at a total flow rate of 15 ml/min, rotor speed of 900g and at room temperature. After 100 ml of eluate are collected, the flow rate is increased to 25 ml/min. With the rotor speed held constant, the flow rates are sequentially increased to 29 ml/min, 33 ml/min, and 37 ml/min, collecting 200 ml with each increment. The cells that remain in the chamber are captured by turning the rotor off and flushing the chamber with 100 ml of elutriation media. Each cell fraction is washed and centrifuged at 300g for 10 minutes. Suitable fractions are collected, viability is determined by trypan blue dye exclusion and cell recoveries are determined with cell counter (Coulter Electronics, Hialeah, FL).

Alternatively liver cells are not separated by density gradient separation and are suspended in phosphate buffered saline (PBS), pH 7. 4, containing 5% fetal calf serum, 0.01% EDTA wt/vol., and 1.0 g/l D-glucose, and injected into a Beckman counterflow centrifugal elutriation system at 10°C at a rotor speed of 1, 950 rpm using a JA-17 rotor and standard separation chamber (Beckman Instruments) and samples are eluted at flow rates between 12 and 14 ml/min.

The cells obtained in the suitable fractions generally have cell diameters in a range of 5 to 15 microns, preferably 8.0 to 9. 4 microns; the majority of the cells had diameters that fell within a range of 8.3 to 9.2 microns. These diameters are measured according to techniques known in the art. If necessary, further selection either positive or negative, based on cell markers is carried out.

A variety of other antibodies known to those of skill in the art may be used alone or in combination with liver progenitor markers. The choice will depend upon the cell type desired to be isolated or enriched and include, but are not limited to, antibodies specific to hematopoietic and lymphoid antigens such as, anti-CD2, anti-CD2R, anti-CD3, anti-CD4, anti- CD5 and anti-CD8 specific for T cells ; anti-CD6 specific for T-cell subset and B-cell subset ; anti-CD7 specific for major T-cell subset; anti-CD 12, anti-CD 19 and anti-CD20, anti-CD72, anti-CDw78, specific for B cells ; anti-CD13 and anti-CD14 specific for monocytes; anti-CD16 and anti-CD56 specific for natural killer cells ; anti-CD41 for platelets; anti-CD1a, CD1b and CD1c specific for cortical thymocytes and Langerhans cells ; anti-CD9 specific for pre-B-cells, monocytes & platelets ; anti-CD 10 specific for lymphoid progenitor cells, C-All and granuloytes ; anti-CD11 a specific for leucocytes ; anti-CD 11 b specific for granulocytes, monocytes and natural killer cells; anti-CD11c specific for monocytes, granulocytes, natural killer cells and hairy cell leukaemia ; anti-CD15 specific for granulocytes; anti-CDw17 specific for granulocytes, monocytes and platelets ; anti-CD18 specific for leucocytes ; anti-CD21 specific for mature B-cells; anti-CD22 specific for B-cells cytoplasm and mature B-cells; anti-CD23 specific for activated B-cells; anti-CD24 specific for B-cells and granulocytes ; anti- CD25 and anti-CD26 specific for activated T-and B-cells and activated macrophages ; anti- CD27 and anti-CD28 specific for major T-cell subset; anti-CD30 specific for activated T-and B-cells and Sternberg Reed cells; anti-CD31 specific for platelets, monocytes/macrophages, granulocytes and B-cells; anti-CDw32 specific for macrophages, granulocytes, B-cells and eosinophils; anti-CD33 specific for monocytes, myeloid progenitor cells and myeloid leukaemias ; anti-CD34 specific for haematopoietic precursor cells ; anti-CD35 specific for granulocytes, monocytes, B-cells, some NK cells, and erythrocytes ; anti-CD36 specific for monocytes/macrophages and platelets ; anti-CD37 specific for mature B-cells ; anti-CD38 specific for plasma cells, thymocytes and activated T-cells ; anti-CD39 specific for mature B- cells ; anti-CD40 specific for B-cells and carcinoma ; anti-CD42 and 42b specific for platelets and megakaryocytes ; anti-CD43 specific for leucocytes except circulating B-cells ; anti-CD44 specific for leucocytes and red cells ; anti-CD45 specific for leucocytes ; anti-CD45RO specific for T-cells, B-cells subset, monocytes and macrophages ; anti-CD45RA specific for B-cells, monocytes and T-cell subset; anti-CD45RB specific for B-cells, T-cells subset, monocytes macrophages and granulocytes ; anti-CD46, CD55, CD58 and CD59 specific for hemopoietic and non-hemopoietic cells ; anti-CD47 specific for all cell types; anti-CD48 specific for leucocytes and neutrophils; anti-CDw49b specific for platelets, activated and long-term cultivated T-cells ; anti-CDw49d specific for monocytes, T-cells and B-cells; anti-CDw49f specific for platelets and megakaryocytes; anti-CDw50 and CDw52 specific for leucocytes; anti-CD51 specific for platelets; anti-CD53 specific for leucocytes including normal and neoplastic plasma cells; anti-CD54 specific for endothelial cells; anti-CDw60 specific for T-cells subset and platelets; anti-CD61 specific for platelets & megakaryocytes; anti-CD62 specific for activated platelets ; anti-CD63 specific for activated platelets, monocytes/macrophages ; anti-CD64 specific for monocytes (upregulated interferon. gamma.); anti-CDw65 specific for granulocytes and heterogenons reactivity with monocytes; anti-CD66 & 67 specific for granulocytes; anti-CD68 specific for monocytes and macrophages; anti-CD69 specific for activated B-and T-cells, activated macrophages, and natural killer cells ; anti-CDw70 specific for activated T-and B-cells, Sternberg-Reed cells, and anaplastic large cell lymphoma ; anti-CD71 specific for activated T-and B-cells, macrophages, proliferating cells ; anti-CD73 specific for B-cell subset and T-cell subset; anti-CD74 specific for B-cells and monocytes/macrophages; anti-CDw75 specific for mature B-cells; anti-CD76 specific for mature B-cells and T-cell subset; anti-CD77 specific for follicular center B-cells; antibodies to cytokines and growth factors (*e.g.* IL1-IL13, EGF, IGF I and 11, TGF-. alpha. and. beta., TNF- . alpha. and .beta., FGF, NGF, CIF, IFN-. alpha. and. beta., CSF's) ; viral antigens (*e.g.* Hepatitis B virus envelope proteins or HIV envelope proteins), hormones, cellular or tumor associated antigens or markers, adhesion molecules, hemostasis molecules, and endothelial cells. Other markers and enrichment procedures are equally suitable such as disclosed in U. S. Pat. No. 5,840,502 .

### Example 13

### Bioreactor

A high performance bioreactor (HPBR) is employed to cultivate human hepatocyte progenitors and their progeny. This process will provide a large number of cells useful for further medical purposes or the bioreactor by itself serves as a production unit for biologically useful cell-secreted proteins and factors that may include, but are not limited to hepatocyte growth factor (HGF), insulin-like growth factor-I and II (IGF-I and II), epidermal growth factor (EGF), type a and type b transforming growth factor (TGF-a and TGF-beta), nerve growth factor (NGF), fibroblast growth factor (FGF), platelet-derived growth factor (PDGF), sarcoma growth factor (SGF), granulocyte macrophage colony stimulating growth factor (GM- CSF), vascular endothelial growth factor (VEGF), prolactin and growth hormone releasing factor (GHRF) and various hemopoietic growth factors such as interleukins (IL) IL-1, IL-2, IL- 3, IL-4, IL-5, IL-6, IL-7, IL-8, IL-10, IL-11, etc., erythroid differentiation factor (EDF) or follicle-stimulating hormone releasing protein (FRP), inhibin, stem cell proliferation factor (SCPF) and active fragments, subunits, derivatives and combinations of these proteins among many others known in the art. Generally, as used herein, these cellular factors refer to a secreted protein which is selected from the group consisting of a cytokine, a lymphokine, an interleukine, a colony-stimulating factor, a hormone, a chemotactic factor, an anti-chemotactic factor, a coagulation factor, a thrombolytic protein, a complement protein, an enzyme, an immunoglobulin, and an antigen. Among such biologically active proteins one skilled in the art may select Factor VIII, Factor IX, Factor VII, erythropoietin, alpha-1-antitrypsin, calcitonin, growth hormone, insulin, low density lipoprotein, apolipoprotein E, IL-2 receptor and its antagonists, superoxide dismutase, immune response modifiers, parathyroid hormone, the interferons (IFN alpha, beta, or gamma), nerve growth factors, glucocerebrosidase, colony stimulating factor, interleukins (IL) 1 to 15, granulocyte colony stimulating factor (G-CSF), granulocyte, macrophage-colony stimulating factor (GM-CSF), macrophage-colony stimulating factor (M-CSF), fibroblast growth factor (FGF), platelet-derived growth factor (PDGF), adenosine deaminase, insulin-like growth factors (IGF-1 and IGF-2), megakaryocyte promoting ligand (MPL), thrombopoietin, or combination thereof.

Without limiting to this particular protocol of growing cells in a bioreactor, other well-known in the art procedures are equally suitable and can be easily adopted from published U. S. Pat. Nos. 6,001,585; 5,998,184; 5,846,817; 5,622,857; 5,571,720; 5,563,068; 5,512,474 ; 5,443,985; 5,342 781; 5,330,915; 5,320,963; 5,202,254; 4,833,083; and 4, 760,028 .

The instant device contains 450 10 kD cellulose fibers 540 polypropylene fibers and details on other parameters are found for example in U. S. Pat. No. 5,622,857 . Cells are isolated as disclosed above. All necessary materials are obtained from either Sigma Chemical Co. or Life Technologies. Attachment media for long-term culture media is as follows: RPMI 1640 (500 mL); 50 mL (10%) FBS; 4 mM L- glutamine; 1x Penicillin/streptomycin ; Gentamicin; 15 mM HEPES ; 10 mU/mL Insulin ; 10 mU/mL transferrin ; selenium. The HPBR system is flushed with media for one day before attachment media is applied. 500 mg of preswollen Cytodex 3 microcarriers are inoculated in the inner annular space of the HPBR. The oxygenator fibers cradled the microcarriers and prevented them from distributing throughout the ECS. Viable human hepatic progenitors are also inoculated into the inner annular space, and the device rocked and rotated by hand to achieve uniform mixing of cells and microcarriers. Assuming that the progenitors and progeny are between 10-20 µm diameter, the cell-to-microcarrier inoculum ratio is about 500. The apparent viscosity of cells and microcarriers increases rapidly, indicating that cell-to-microcarrier and cell-to-cell attachments are proceeding rapidly and normally. Within a 2-3 minutes of this mixing a discrete gel of cells and microcarriers is formed in the inner annular space. Following an overnight incubation at 37 °C in attachment media (in a stationary position), the media is changed to long-term culture media (2 L). These volumes are not limiting in any way as one skilled in the art can scale easily the production to the desired level. The hepatocytes are cultured for 5 weeks, with fresh media applied to the system weekly. The metabolic function of the cells is monitored by testing daily samples. After 5 weeks, >90% recovery of viable cells and microcarriers is achieved by the following procedure: 0.1 % collagenase in PBS mixed with 0.44 mL (0.23 M) EDTA is used to flush the ECS and the HPBRr incubated for 10 minutes; the content of the ECS is expelled with sterile air from a syringe barrel; this process is repeated with long-term culture media and the materials collected washed and separated.

The HPBR is equally suitable in the cultivation and genetic transformation of cells (*e.g.*, HGF gene expression). The following is a genetic non-viral protocol for anchorage dependent cells (*e.g.*, SW 480 P3; ATCC #CCL228), that can be appropriately modified and optimized from published procedures using culture wells and dishes, by those skilled in the art. Media fiber with 10 kD properties are preferred in the HPBR. The bioreactor is operated in much the same manner as described supra. Cytodex 1 microcarrier (Pharmacia, sold by Sigma Chemical Co.) are widely use for culturing anchorage dependent cells. A broad range of cell densities can be inoculated into the ECS of the HPBR, ranging from: 1x10⁴ to 1x10¹⁵ cells or higher as desired. The recommended cell-to-microcarrier inoculum ratio is in the range of about 10, although one skilled in the art can modify this as desired. The device is gently rotated throughout the experiment at about 10 cpm (or greater). After culturing the cells for about one day (or more, depending on the specific cell), optimal confluence is attained to obtain efficient transfection. The cell-to-microcarrier inoculation ratio is adjustable to positively impact this time frame for therapeutic and economic efficiency. On the day of the transfection, prepare the DNA plasmid solution (*e.g.*, pCMV), and cationic lipid solution (*e.g.*, LIPOFECTIN Reagent, Life Technologies). These reagents must be serum free, even if the overall process requires the presence of serum. Mix appropriate quantities of DNA and lipid solutions, then inject the mixture into the ECS of the device. After about a few (or even several) hours of transfection, resume use of serum, if appropriate, and continue to culture cells as before for about a few days. Longer periods may be used when expanding permanently transformed cells. Harvest cells in a manner similar to that described previously.

### Example 14

### Artificial liver

As an extension of above example one skilled in the art can easily adopt the bioreactor as an extracorporeal hepatic support system. Xenotransplantation (the transplantation of organs between species) may help alleviate the shortage of donor livers by using animal organs. A potential danger of transplanting animal organs into humans, however, is that viruses that infect the donor animals may infect the recipients. As the organ transplant recipients would be taking drugs to surpress the immune system and prevent organ rejection, they may be unable to fight off the infecting animal virus. In an even more frightening scenario, the animal virus may mutate in the infected host into a form that can infect human contacts with normal immune systems. As a result, a new pathogenic human virus may arise. A favorite animal species for human organ transplantation is the pig and also primates. Nevertheless it is clear that if human cell-based artificial liver is available, it would be preferable to animal livers.

After the desired time in culture mature hepatocytes and/or biliary cells derived from a population enriched in liver progenitors are obtained. Routinely 2 to 5 billion cells of high (over 80%) viability are obtained. In general the culture medium used is the hormone- supplemented Waymouth medium. To accommodate 2 to 5 billion cells, the bioreactor is scaled up to two containment vessels, each with an internal diameter of 40 mm and a height of 100 mm. In this particular situation glass beads of approximately 2 mm in diameter and a total volume of 250 ml per containment vessel are used. Medium is supplied at a recycle rate of 360 ml/min. The high viability of the hepatocytes is evidenced by the stable oxygen consumption rate. The bioreactor is then attached to an ahepatic human recipient whose liver is removed by surgery due to total hepatic failure. Similarly, the bioreactor is attached to a human subject with a dysfunctional liver. A skilled artisan will know the procedures for attaching the bioreactor as an extracorporeal hepatic support system or will know alternative means known in the art such as disclosed for example in the U. S. Pat. Nos. 6,008,049 ; 5,981 211; 5,976, 70; 5,891,713 ; 5,827,729; 5 643,794; 5,622,857; 5,605,835 and 5,270,192. It is evident from such references that donor artificial liver cells are not necessarily limited to human species and cross-species use of such cells is now possible. F or example, liver cell from pigs or primates are equally suitable for human use. It is equally evident that the methods and compositions of the instant invention permit preparation of human liver cells for use in cell therapy or extracorporeal liver therapy, with all the advantages attendant thereto.

Blood from the left femoral artery is directed into a Minntech hemoconcentrator. A 12 fringe elecath canula is inserted into the femoral artery and connected to a 1/4"PVC tubing to the hemoconcentrator. The hemoconcentrator separated the blood into a cell free ultrafiltrate fraction, and a blood cell fraction. The blood cell fraction is returned to the femoral vein via a similar tubing. The ultrafiltrate exited the hemoconcentrator via a 1/4VC tubing and entered the hepatocyte bioreactor system with the flow rate adjusted to 40 ml/min. using a roller pump. After perfusion through the bioreactor, the ultrafiltrate is returned to the patient via the left jugular vein. To demonstrate the provision of extracorporeal hepatic metabolism, two different chemicals known to be metabolized by the liver, 7-ethoxycoumarin and lidocaine, are administered into the ultrafiltrate at the inlet of the bioreactor. The respective metabolites, 7-OH-coumarin and monoethylglycinexylidide (MEGX), are measured at the outlets of the bioreactors before the ultrafiltrate is returned to the patient. Significant metabolism of both 7-ethoxycoumarin and lidocaine are observed. The results therefore demonstrate the application of the bioreactor as a support system, providing extracorporeal hepatic metabolism. The separation of the blood cells from the plasma minimizes immunological reaction of the recipient to the foreign hepatocytes. Hepatic progenitors and their progeny are thus useful in the bioreactor to provide extracorporeal hepatic support.

### Example 15

### Exon 1-encoded Peptides and use as Antigens

Short peptides corresponding to the exon 1 of alpha-fetoprotein are used to unambiguously distinguish alpha-fetoprotein in various cell lineages by evaluating expression with specific antibodies. The exon 1-encoded peptide sequence is: SEQ. ID 14 MKWVESIFLIFLLNFTESRTLHRNEYGI These amino acids can be also represented by an alphabetical string such as ABCDEFGHIJKLMNOPRSTUVWXYZ such that letter A from this string starts from position M, K, W, V, E, S, I, F, L, I, F, L, L, or N of the peptide. Peptides of the exon 1-encoded sequence and between four and twelve amino acid residues in length are conjugated to a macromolecule to produce an antigen. The peptide is optionally linked to the macromolecule by a spacer of from two to eight carbon atoms in length. The macromolecule is albumin, hemocyanin, casein, ovalbumin, or polylysine. Suitable peptides include the peptides in the table and analogs with at least 80% homology or standard substitute amino acids. The following is the example one skilled in the art construes to obtain desired peptide sequence and length according to specific needs:
A--B--C--D--E--F--G--H--I--J--K--L--M--N,
A--B--C--D--E--F--G--H--I--J--K--L--M,
A--B--C--D--E--F--G--H--I--J--K--L,
A--B--C--D--E--F--G--H--I--J--K,
A--B--C--D--E--F--G--H--I--J,
A--B--C--D--E--F--G--H--I--J,
A--B--C--D--E--F--G--H-I,
A--B--C--D--E--F--G--H,
A--B--C--D--E--F--G,
A--B--C--D--E--F--G,
A--B--C--D--E--F,
A--B--C--D--E,
A--B--C--D,
B--C--D--E--F--G--H--I--J--K--L--M--N,
B--C--D--E--F--G--H--I--J--K--L--M,
B--C--D--E--F--G--H--I--J--K--L,
B--C--D--E--F--G--H--I--J--K,
B--C--D--E--F--G--H--I--J,
B--C--D--E--F--G--H--I,
B--C--D--E--F--G--H,
B--C--D--E--F--G,
B--C--D--E--F
B--C--D--E
C--D--E--F--G--H--I--J--K--L--M--N,
C--D--E--F--G--H--I--J--K--L--M,
C--D--E--F--G--H--I--J--K--L,
C--D--E--F--G--H--I--J,
C--D--E--F--G--H--I,
C--D--E--F--G--H,
C--D--E--F,
D--E--F--G--H--I--J--K--L--M--N,
D--E--F--G--H--I--J--K--L--M,
D--E--F--G--H--I--J--K--L,
D--E--F--G--H--I--J--K,
D--E--F--G--H--I--J,
D--E--F--G--H--I--J--K--L--M--N,
D--E--F--G--H--I,
D--E--F--G,
E--F--G--H--I--J--K--L--M--N,
E--F--G--H--I--J--K--L--M,
E--F--G--H--I--J--K--L,
E--F--G--H--I--J--K,
E--F--G--H--I--J,
E--F--G--H--I,
E--F--G--H,
F--G--H--I--J--K--L--M--N,
F--G--H--I--J--K--L--M,
F--G--H--I--J--K--L,
F--G--H--I--J--K,
F--G--H--I--J,
F--G--H--I,
G--H--I--J--K--L--M--N,
G--H--I--J--K--L--M,
G--H--I--J--K--L,
G--H--I--J--K,
G--H--I--J,
H--I--J--K--L--M--N,
H--I--J--K--L--M,
H--I--J--K--L,
H_-I--J--K,
I--J--K--L--M--N,
I--J--K--L--M,
I--J--K--L,
J--K--L--M--N,
J--K--L--M,
K--L--M--N and the like,
Wherein any of A-B--C--D--E--F--G--H--I--J--K--L--M-or N, can be nonpolar amino acids (hydrophobic)
Such as glycine Gly G
alanine Ala A
valine Val V
leucine Leu L
isoleucine Ile I
methionine Met M
phenylalanine Phe F
tryptophan Trp W
proline Pro P,
or polar (hydrophilic)
serine Ser S
threonine Thr T
cysteine Cys C
tyrosine Tyr T
asparagines Asn N
glutamine Gln Q
or electrically charged (negative)
aspartic acid Asp D
glutamic acid Glu E
or electrically charged (positive)
lysince Lys K
arginine Arg R
histidine His H
or absent. The string can be composed of acceptable amino acid substitutes or salts therof. The most frequently amino acid substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, and vice versa.

## Claims

1. A method of providing a composition comprising an enriched population of human hepatic progenitors, the method comprising:
(a) providing a substantially single cell suspension of extracted human liver tissue;
(b) debulking the suspension based on cell size, buoyant density, or a combination thereof to remove mature cells while retaining immature cells; and
(c) subjecting the debulked suspension to a positive immunoselection comprising selecting those cells which express CD14, CD34, CD38 or combinations thereof, and/or a negative immunoselection comprising removing those cells which express CD45, glycophorin A or a combination thereof, such that a mixture of cells is provided, which mixture of cells is comprised of an enriched population of human hepatic progenitors and expresses alpha-fetoprotein, albumin, or both.

2. The method of claim 1 in which the liver tissue is obtained from a neonate, an infant, a child, a juvenile, or an adult.

3. The method of claim 1 in which the immature cells have a diameter less than 15 microns.

4. The method of claim 1 in which the enriched population comprises human diploid liver cells.

5. The method of claim 1, in which the debulking comprises separation according to cell size, buoyant density, or a combination thereof.

6. The method of claim 1 in which the debulking step comprises centrifugal elutriation, density gradient centrifugation, panning, affinity chromatography, tagging with fluorescent labels, countercurrent fluid flow, continuous-flow centrifugation, zonal centrifugation, use of magnetic beads, or combinations thereof.

7. The method of claim 1 which further comprises selective lysis of the mature cells.

8. The method of claim 3 in which the immature cells have a diameter between 5 and 15 microns.

9. The method of claim 3 in which the immature cells have a diameter between 8 and 9.4 microns.

## Patentansprüche

1. Verfahren, um eine Zusammensetzung zur Verfügung zu stellen, die eine angereicherte Population von humanen hepatischen Progenitoren aufweist, bei dem man
(a) eine Suspension von im Wesentlichen Einzelzellen aus extrahiertem humanem Lebergewebe zur Verfügung stellt,
(b) die Suspension, basierend auf Zellgröße, Schwimmdichte oder einer Kombination davon auseinandernimmt, um reife Zellen zu entfernen, während unreife Zellen zurückgehalten werden und
(c) die auseinandergenommene Suspension einer positiven Immunoselektion unterzieht, bei der solche Zellen ausgewählt werden, die CD14, CD34, CD38 oder Kombinationen davon exprimieren, und/oder einer negativen Immunoselektion unterzieht, bei der solche Zellen entfernt werden, die CD45, Glycophorin A oder eine Kombination davon exprimieren, sodass eine Mischung von Zellen zur Verfügung gestellt wird, die eine angereicherte Population von humanen hepatischen Progenitoren aufweist und alpha-Fetoprotein, Albumin oder beides exprimiert.

2. Verfahren nach Anspruch 1, wobei das Lebergewebe aus einem Neugeborenen, einem Säugling, einem Kind, einem Jugendlichen oder einem Erwachsenen erhalten wird.

3. Verfahren nach Anspruch 1, wobei die unreifen Zellen einen Durchmesser von weniger als 15 Mikrometer haben.

4. Verfahren nach Anspruch 1, wobei die angereicherte Population humane diploide Leberzellen aufweist.

5. Verfahren nach Anspruch 1, wobei das Zerkleinern das Auftrennen nach Zellengröße, Schwimmdichte oder eine Kombination davon aufweist.

6. Verfahren nach Anspruch 1, wobei der Zerkleinerungsschritt zentrifugale Elutriation, Dichtegradientenzentrifugation, Schwenken, Affinitätschromatographie, Markieren mit Fluoreszenzmarkern, Gegenstromfluidfluss, Durchlaufzentrifugation, Zonenzentrifugation, Verwendung von magnetischen Kügelchen oder Kombinationen davon aufweist.

7. Verfahren nach Anspruch 1, welches weiterhin selektive Lyse der reifen Zellen aufweist.

8. Verfahren nach Anspruch 3, wobei die unreifen Zellen einen Durchmesser zwischen 5 und 15 Mikrometer haben.

9. Verfahren nach Anspruch 3, wobei die unreifen Zellen einen Durchmesser zwischen 8 und 9,4 Mikrometer haben.

## Revendications

1. Procédé de fourniture d'une composition comprenant une population enrichie de progéniteurs hépatiques humains, le procédé comprenant:
(a) fournir une suspension sensiblement de cellules individuelles de tissu hépatiques humain extrait ;
(b) traiter la suspension sur la base de la taille des cellules, de la densité de flottaison, ou d'une combinaison de celles-ci pour retirer les cellules matures tout en conservant les cellules immatures ; et
(c) soumettre la suspension traitée à une immunosélection positive comprenant la sélection des cellules qui expriment CD14, CD34, CD38 ou une combinaison de ceux-ci, et/ou une immunosélection négative comprenant le retrait des cellules qui expriment CD45, la glycophorine A ou une combinaison de ceux-ci, de sorte qu'un mélange de cellules est fourni, lequel mélange de cellules comprend une population enrichie de progéniteurs hépatiques humains et exprime l'alpha-fétoprotéine, l'albumine ou les deux.

2. Procédé selon la revendication 1 où le tissu hépatiques est obtenu auprès d'un nouveau-né, d'un nourrisson, d'un enfant, d'un jeune ou d'un adulte.

3. Procédé selon la revendication 1 où les cellules immatures ont un diamètre inférieur à 15 micromètres.

4. Procédé selon la revendication 1 où la population enrichie comprend des cellules hépatiques diploïdes humaines.

5. Procédé selon la revendication 1 où le traitement comprend la séparation selon la taille des cellules, la densité de flottaison, ou une combinaison de celles-ci.

6. Procédé selon la revendication 1 où l'étape de traitement comprend l'élutriation centrifuge, la centrifugation en gradient de densité, le lavage, la chromatographie d'affinité, le marquage avec des marqueurs fluorescents, l'écoulement fluide à contre-courant, la centrifugation à écoulement continu, la centrifugation zonale, l'utilisation de billes magnétiques, ou une combinaison de ceux-ci.

7. Procédé selon la revendication 1 qui comprend en outre la lyse sélective des cellules matures.

8. Procédé selon la revendication 3 où les cellules immatures ont un diamètre entre 5 et 15 micromètres.

9. Procédé selon la revendication 3 où les cellules immatures ont un diamètre entre 8 et 9,4 micromètres.


### SEQUENCE LISTING

<110> Reid, Lola
<120> Human Liver Progenitors
<130> 113918.101
<140> New
   <141> 2000-01-19
<160> 1
<170> PatentIn Ver. 2.1
<210> 1
   <211> 20
   <212> DNA
   <213> Homo sapiens
<400> 1
   accatgaagt gggtggaatc 20

### SEQUENCE LISTING

<110> Reid, Lola
<120> Human Liver Progenitors
<130> 113918.101 Sequence 2
<140> New
   <141> 2000-01-19
<160> 1
<170> PatentIn Ver. 2.1
<210> 1
   <211> 20
   <212> DNA
   <213> Homo sapiens
<400> 1
   cctgaagact gttcatctcc 20

### SEQUENCE LISTING

<110> Reid, Lola
<120> Human Liver Progenitors
<130> 113918.101 Sequence 3
<140> New
   <141> 2000-01-19
<160> 1
<170> PatentIn Ver. 2.1
<210> 1
   <211> 20
   <212> DNA
   <213> Homo sapiens
<400> 1
   taaaccctgg tgttggccag 20

### SEQUENCE LISTING

<110> Reid, Lola
<120> Human Liver Progenitors
<130> 113918-101 Sequence 4
<140> New
   <141> 2000-01-19
<160> 1
<170> PatentIn Ver. 2.1
<210> 1
   <211> 22
   <212> DNA
   <213> Homo sapiens
<400> 1
   atttaaactc ccaaagcagc ac 22

### SEQUENCE LISTING

<110> Reid, Lola
<120> Human Liver Progenitors
<130> 113918.101 Sequence 5
<140> New
   <141> 2000-01-19
<160> 1
<170> PatentIn Ver. 2.1
<210> 1
   <211> 25
   <212> DNA
   <213> Homo sapiens
<400> 1
   cttccatatt ggattcttac caatg 25

### SEQUENCE LISTING

<110> Reid, Lola
<120> Human Liver Progenitors
<130> 113918.101 Sequence 6
<140> New
   <141> 2000-01-19
<160> 1
<170> PatentIn Ver. 2.1
<210> 1
   <211> 22
   <212> DNA
   <213> Homo sapiens
<400> 1
   ggctaccata ttttttgccc ag 22

### SEQUENCE LISTING

<110> Reid, Lola
<120> Human Liver Progenitors
<130> 113918.101 Sequence 7
<140> New
   <141> 2000-01-19
<160> 1
<170> PatentIn Ver. 2.1
<210> 1
   <211> 22
   <212> DNA
   <213> Homo sapiens
<400> 1
   ctacctgcct ttctggaaga ac 22

### SEQUENCE LISTING

<110> Reid, Lola
<120> Human Liver Progenitors
<130> 113918-101 Sequence 8
<140> New
   <141> 2000-01-19
<160> 1
<170> PatentIn Ver. 2.1
<210> 1
   <211> 21
   <212> DNA
   <213> Homo sapiens
<400> 1
   gagatagcaa gaaggcatcc c 21

### SEQUENCE LISTING

<110> Reid, Lola
<120> Human Liver Progenitors
<130> 113918-101 Sequence 9
<140> New
   <141> 2000-01-19
<160> 1
<170> PatentIn Ver. 2.1
<210> 1
   <211> 24
   <212> DNA
   <213> Homo sapiens
<400> 1
   aaagaattaa gagaaagcag cttg 24

### SEQUENCE LISTING

<110> Reid
<120> Human Liver Progenitors
<130> Sequence 10
<140>
   <141>
<160> 1
<170> PatentIn Ver. 2.1
<210> 1
   <211> 21
   <212> DNA
   <213> Homo sapiens
<400> 1
   ggcacaatga agtgggtaac c 21

### SEQUENCE LISTING

<110> Reid
<120> Human Liver Progenitors
<130> Sequence 11
<140>
   <141>
<160> 1
<170> PatentIn Ver. 2.1
<210> 1
   <211> 22
   <212> DNA
   <213> Homo sapiens
<400> 1
   ccataggttt cacgaagagt tg 22

### SEQUENCE LISTING

<110> Reid
<120> Human Liver Progenitors
<130> Sequence 12
<140>
   <141>
<160> 1
<170> PatentIn Ver. 2.1
<210> 1
   <211> 21
   <212> DNA
   <213> Homo sapiens
<400> 1
   gccagtaagt gacagagtca c 21

### SEQUENCE LISTING

<110> Reid
<120> Human Liver Progenitors
<130> Sequence 13
   <140>
   <141>
<160> 1
<170> PatentIn Ver. 2.1
<210> 1
   <211> 23
   <212> DNA
   <213> Homo sapiens
<400> 1
   ttataagcct aaggcagctt gac 23

### SEQUENCE LISTING

<110> Reid
<120> Human Liver Progenitors
<130> Sequence 14
   <140>
   <141>
<160> 1
<170> Patent In Ver. 2.1
<210> 1
   <211> 28
   <212> PRT
   <213> Homo sapiens
<400> 1
   Met Lys Trp Val Glu Ser Ile Phe Leu Ile Phe Leu Leu Asn Phe Thr
     1               5                  10                  15
   Glu Ser Arg Thr Leu His Arg Asn Glu Tyr Gly Ile
                20                  25